# EUROPEAN PATENT APPLICATION

(11) **EP 4 603 971 A1**
(43) Date of publication of application: **20.08.2025**
(21) Application number: 24802758.3
(22) Date of filing: 23.04.2024
(51) Int. Cl.: G06F 9/451

(54) **DISPLAY METHOD AND ELECTRONIC DEVICE**

(30) Priority: 06.05.2023 CN 202310511093; 29.06.2023 CN 202310789242
(71) Applicant: Huawei Technologies Co., Ltd., Shenzhen, Guangdong 518129 (CN)
(72) Inventor: CHEN, Yumei, Shenzhen, Guangdong 518129 (CN); JIA, Zheng, Shenzhen, Guangdong 518129 (CN); MAO, Chenyang, Shenzhen, Guangdong 518129 (CN); LUO, Long, Shenzhen, Guangdong 518129 (CN); CHEN, Qinsheng, Shenzhen, Guangdong 518129 (CN)
(74) Representative: Goddar, Heinz J.
(86) International application number: PCT/CN2024/089365
(87) International publication number: WO 2024/230473

(57) **Abstract**

A display method and an electronic device are disclosed. The method includes: displaying a first interface of a first application, where the first interface includes a first application card, a second application card is displayed in the first application card, and measurement information of a physiological parameter is not displayed in the second application card; obtaining measurement information of at least one type of physiological parameter; and displaying a second interface of the first application, where the second interface includes a new first application card, the measurement information is displayed in the new first application card, the new first application card is displayed above the first application card, and the second application card is not displayed in the first application card. In this way, a user can quickly and conveniently obtain a measurement result of the physiological parameter, thereby improving the user's efficiency of obtaining information.

## Description

This application claims priorities to Chinese Patent Application No. 202310511093.3, filed with the China National Intellectual Property Administration on May 6, 2023 and entitled "CARD DISPLAY METHOD AND ELECTRONIC DEVICE", and to Chinese Patent Application No. 202310789242.2, filed with the China National Intellectual Property Administration on June 29, 2023 and entitled "DISPLAY METHOD AND ELECTRONIC DEVICE", both of which are incorporated herein by reference in their entireties.

### TECHNICAL FIELD

This application relates to the field of computer technologies, and in particular, to a display method and an electronic device.

### BACKGROUND

Currently, after measuring physiological parameters by using a wearable device, a user may view measurement results of the physiological parameters by using an electronic device. However, because there are various types of measured physiological parameters, for example, including but not limited to blood pressure, blood glucose, respiration, and a heart rate, when the user wants to view a measurement result of any one of the physiological parameters, the user needs to start a health app and perform a series of operations (for example, perform page switching a plurality of times and drag a scroll bar on a page). Consequently, cumbersome user operations decrease the user's efficiency of obtaining information.

### SUMMARY

This application discloses a display method and an electronic device, so that a user can quickly and conveniently obtain a measurement result of a physiological parameter, thereby improving the user's efficiency of obtaining information.

According to a first aspect, an embodiment of this application provides a display method, applied to a first device, where a first application is installed on the first device. The method includes: displaying a first interface of the first application, where the first interface includes a first application card, a second application card is displayed in the first application card, and measurement information of a physiological parameter is not displayed in the second application card; obtaining measurement information of at least one type of physiological parameter; and displaying a second interface of the first application, where the second interface includes a new first application card, the measurement information is displayed in the new first application card, the new first application card is displayed above the first application card, and the second application card is not displayed in the first application card.

In the foregoing method, the first interface of the first application includes the first application card, the second application card is displayed in the first application card, and the measurement information of the physiological parameter is not displayed in the second application card. After the first device obtains the measurement information of the at least one type of physiological parameter of the user, the new first application card may be generated based on the measurement information, and the new first application card is displayed on the top of the second interface of the first application. In other words, the new first application card is automatically displayed on the top, and the user may obtain the measurement information of the at least one type of physiological parameter based on the new first application card displayed on the top of the second interface, so that operations such as starting a plurality of apps, switching pages a plurality of times, and dragging and sliding a scroll bar on a page a plurality of times do not need to be performed. In this manner, the user can quickly and conveniently obtain the measurement information of the physiological parameter, operations that need to be performed by the user to obtain the measurement information are reduced, and user operations are greatly reduced especially when there are a large quantity of physiological parameters. This effectively improves the user's efficiency of obtaining information, and improves user experience.

In a possible implementation, the new first application card is displayed in the second interface in a first display order, and the first display order is related to at least one of the following: generation time of the new first application card, obtaining time of the measurement information of the physiological parameter indicated by the new first application card, and a risk level of the measurement information of the physiological parameter indicated by the new first application card.

In some examples, the first display order is related to the generation time of the new first application card. For example, new first application cards are sequentially sorted from bottom to top in the second interface based on generation time of the new first application cards from early to late. It may be understood that the later the generation time, the upper the corresponding application card is displayed.

In some examples, the first display order is related to the obtaining time of the measurement information of the physiological parameter indicated by the new first application card. For example, the new first application cards are sequentially sorted from bottom to top in the second interface based on the obtaining time of the measurement information from early to late.

In a possible implementation, the first display order is related to the risk level of the measurement information of the physiological parameter indicated by the new first application card, the new first application card includes a third application card and a fourth application card, the third application card is displayed above the fourth application card, and a risk level of measurement information of a physiological parameter indicated by the third application card is higher than a risk level of measurement information of a physiological parameter indicated by the fourth application card.

In the foregoing method, the new first application cards are displayed in the first display order, so that the user can conveniently view measurement results of physiological parameters. For example, a newest (latest) measurement result is displayed on the top, so that the user can view newest measurement information in time when starting the first application. In addition, measurement information with a highest risk level (a measurement result with a highest risk level) is displayed on the top, and the user can be notified in time, further improving the user's efficiency of obtaining information.

In a possible implementation, a plurality of application cards are displayed in the new first application card, and the method further includes: receiving a first operation for the plurality of application cards; in response to the first operation, obtaining first information based on measurement information of a plurality of types of physiological parameters respectively indicated by the plurality of application cards; and displaying a third interface of the first application, where the third interface includes the first information.

In the foregoing method, the first device may perform data fusion on measurement data of any plurality of types of physiological parameters (namely, the measurement information of the plurality of types of physiological parameters), to generate fused result data (namely, the first information). The fused result data is more comprehensive, a physical condition of the user can be further analyzed by using the fused result data, especially in a case in which the user may have an abnormal symptom, so that the user can better understand a health status of the user, thereby further improving user experience.

In a possible implementation, the third interface includes a fifth application card, the fifth application card indicates a first physiological parameter, the fifth application card includes the first information, and the first physiological parameter includes the plurality of types of physiological parameters respectively indicated by the plurality of application cards.

In the foregoing method, the first device may fuse the plurality of application cards, and display the fused measurement information (namely, the first information) in a fused/new application card (namely, the fifth application card). The fused measurement information is displayed in the new application card, so that the user can directly view the new application card to learn of application cards corresponding to the fused measurement information. This display manner is simple and easy to understand, and helps the user obtain information.

In a possible implementation, the fifth application card includes the measurement information of the plurality of types of physiological parameters respectively indicated by the plurality of application cards, and the third interface does not include the plurality of application cards.

In the foregoing method, the fused measurement information may be displayed in the new application card (namely, the fifth application card), and the measurement information of the plurality of application cards may be further displayed, so that the user can directly obtain a plurality of types of information from the new application card, thereby improving the user's efficiency of obtaining information.

In a possible implementation, the method further includes: receiving a second operation for the second interface; obtaining second information in response to the second operation, where the second information includes measurement information that is of a physiological parameter indicated by the new first application card and that is obtained at first time, the first time is later than obtaining time of measurement information of a physiological parameter indicated by a sixth application card in the second interface, and the sixth application card is any new application card in the new first application card; displaying first progress information in response to the second operation, where the first progress information indicates progress of obtaining the second information; and updating, based on the second information, the measurement information of the physiological parameter indicated by the new first application card in the second interface for display.

In the foregoing method, the first device may update, in response to a user operation, measurement information corresponding to at least one second application card, and display progress information during the update to the user in a visualized manner, so that the user learns of an update progress status in time, thereby reducing anxiety of the user in a waiting process, and further improving user experience.

In a possible implementation, the obtaining second information includes: sequentially obtaining, in a first preset order, the measurement information of the physiological parameter indicated by the new first application card, where the first preset order is related to a display order of the new first application card in the second interface.

In the foregoing method, when the first device obtains the measurement information of the plurality of types of physiological parameters, because a data size of the measurement information is large, and a Bluetooth transmission rate is low, a rate at which an electronic device obtains information is also low, and the electronic device usually needs to wait for several minutes. Sequentially obtaining the plurality of types of physiological parameters in the first preset order can improve orderliness of data transmission, thereby improving a data transmission rate.

In a possible implementation, the new first application card in the second interface indicates a second physiological parameter, and the method further includes: receiving a third operation for the new first application card; displaying a fourth interface of the first application in response to the third operation, where the fourth interface includes measurement information of the second physiological parameter; receiving a fourth operation for the fourth interface; in response to the fourth operation, if the first device is currently obtaining measurement information of a third physiological parameter, displaying progress of obtaining the measurement information of the third physiological parameter; and in response to the fourth operation, if the first device currently does not obtain measurement information of a physiological parameter, obtaining third information, where the third information is the measurement information that is of the second physiological parameter and that is obtained at second time, and the second time is later than obtaining time of the measurement information of the second physiological parameter in the fourth interface, displaying second progress information, where the second progress information indicates progress of obtaining the third information, and updating, based on the third information, the measurement information of the second physiological parameter in the fourth interface for display.

In the foregoing method, when the first device displays an interface of the physiological parameter, in response to a user operation, if the first device is currently obtaining the measurement information, the first device may display the progress of obtaining the measurement information of the physiological parameter; and if the first device currently does not obtain the measurement information, the first device obtains measurement information of a physiological parameter corresponding to the current interface, and displays progress of obtaining the measurement information of the physiological parameter, to update the measurement information of the physiological parameter. The progress information during the update is displayed to the user in a visualized manner, so that the user learns of an update progress status in time, thereby reducing anxiety of the user in a waiting process, and further improving user experience.

In a possible implementation, the method further includes: displaying a first card in a fifth interface, where the fifth interface is an interface of the first device other than an interface of the first application, and the first card is any application card in the new first application card.

In the foregoing method, the first device may display, in a form of card, measurement information of at least one type of physiological parameter in an interface other than a user interface of an application, for example, a home screen, a leftmost screen, a lock screen interface, or a notification interface. In this way, when the user interface of the application is not displayed, the user can still view the measurement information of the physiological parameter in time, thereby effectively improving the user's efficiency of obtaining information and improving user experience.

In a possible implementation, the method further includes: receiving a fifth operation for the first card; and switching the first card to a second card in response to the fifth operation, where the first card is different from the second card, and the new first application card includes the second card.

In the foregoing method, the first card in the fifth interface may be switched to the second card, to switch to display measurement information of different physiological parameters. In this way, when the user interface of the application is not displayed, the user can still view measurement information of a plurality of types of physiological parameters in time, thereby effectively improving the user's efficiency of obtaining information.

In a possible implementation, the new first application card in the second interface indicates the second physiological parameter, and the method further includes: receiving a sixth operation for the new first application card; and displaying a fifth interface of the first application in response to the sixth operation, where the fifth interface does not include the new first application card, the fifth interface includes the second application card, the second application card indicates the second physiological parameter, and the measurement information of the second physiological parameter is not displayed in the second application card.

In the foregoing method, the first device may delete, in response to a user operation, a new first application card corresponding to any physiological parameter and related data (for example, the measurement information of the second physiological parameter indicated by the new first application card), optionally disable a measurement function of the second physiological parameter, and then switch the new first application card corresponding to the second physiological parameter to the second application card for display, to improve visual differentiation between a physiological parameter for which the measurement function is enabled and a physiological parameter for which the measurement function is not enabled/disabled, facilitate browsing and viewing by the user, and improve the user's efficiency of obtaining information.

In a possible implementation, the obtaining measurement information of at least one type of physiological parameter includes at least one of the following: obtaining the measurement information of the at least one type of physiological parameter from a second application related to the at least one type of physiological parameter; receiving the measurement information that is of the at least one type of physiological parameter and that is sent by a second device, where the measurement information of the at least one type of physiological parameter is obtained by the second device by measuring a user; and obtaining the measurement information of the at least one type of physiological parameter from a cloud.

In the foregoing method, the measurement information of the at least one type of physiological parameter may be obtained from the second application, or may be obtained from the second device, or may be obtained from the cloud. The first device may select an appropriate and convenient manner of obtaining the measurement information based on an actual situation. For example, when the user changes to a new mobile phone, the measurement information of the user may be directly obtained from the cloud, to meet different requirements in different use scenarios, so that use of the user is convenient and user experience is further improved.

In a possible implementation, the method further includes: the second application card in the first interface indicates a fourth physiological parameter, and the obtaining measurement information of at least one type of physiological parameter includes: receiving a seventh operation for the second application card; and; displaying a sixth interface of the first application in response to the seventh operation; receiving an eighth operation for a first control in the sixth interface; and obtaining measurement information of the fourth physiological parameter in response to the eighth operation.

In the foregoing method, the first device may receive the user operation, and obtain the measurement information of the fourth physiological parameter. A manner in which the first device obtains the measurement information is triggered and selected by the user, so that different requirements of the user in different use scenarios are met, use of the user is convenient, and user experience is improved.

According to a second aspect, this application provides an electronic device, including a transceiver, a processor, and a memory. The memory is configured to store a computer program, and the processor invokes the computer program to perform the display method in any possible implementation of the first aspect.

According to a third aspect, this application provides an electronic device, including one or more processors and one or more memories. The one or more memories are coupled to the one or more processors. The one or more memories are configured to store computer program code. The computer program code includes computer instructions, and when the one or more processors execute the computer instructions, the electronic device is enabled to perform the display method in any possible implementation of the first aspect.

According to a fourth aspect, this application provides a computer storage medium. The computer storage medium stores a computer program, and when the computer program is executed by a processor, the display method in any possible implementation of any foregoing aspect is performed.

According to a fifth aspect, this application provides a computer program product. When the computer program product runs on an electronic device, the electronic device is enabled to perform the display method in any possible implementation of the first aspect.

According to a sixth aspect, this application provides an electronic device. The electronic device includes the method or the apparatus described in any implementation of the first aspect of this application. The electronic device is, for example, a chip.

### BRIEF DESCRIPTION OF DRAWINGS

The following describes the accompanying drawings used in this application.
FIG. 1 is a diagram of a hardware structure of an electronic device 100 according to this application;
FIG. 2 is a diagram of a software architecture of an electronic device 100 according to this application;
FIG. 3 is a diagram of an electronic device 200 in an unfolded state according to this application;
FIG. 4(A) to FIG. 11(C) are diagrams of some user interfaces according to this application;
FIG. 12 is a schematic flowchart of a display method according to this application;
FIG. 13 is a schematic flowchart of another display method according to this application;
FIG. 14 is a schematic flowchart of another display method according to this application; and
FIG. 15 is a schematic flowchart of another display method according to this application.

### DESCRIPTION OF EMBODIMENTS

The following describes the technical solutions in embodiments of this application with reference to the accompanying drawings. In descriptions of embodiments of this application, unless otherwise specified, "/" indicates "or". For example, A/B may indicate A or B. The term "and/or" in this specification merely describes an association relationship for describing associated objects, and indicates that three relationships may exist. For example, A and/or B may indicate the following three cases: Only A exists, both A and B exist, and only B exists. In addition, in the descriptions of embodiments of this application, "a plurality of" means two or more.

The terms "first" and "second" mentioned below are merely intended for a purpose of description, and shall not be understood as an indication or implication of relative importance or implicit indication of a quantity of indicated technical features. Therefore, a feature limited by "first" and "second" may explicitly or implicitly include one or more features. In the descriptions of embodiments of this application, unless otherwise specified, "a plurality of" means two or more.

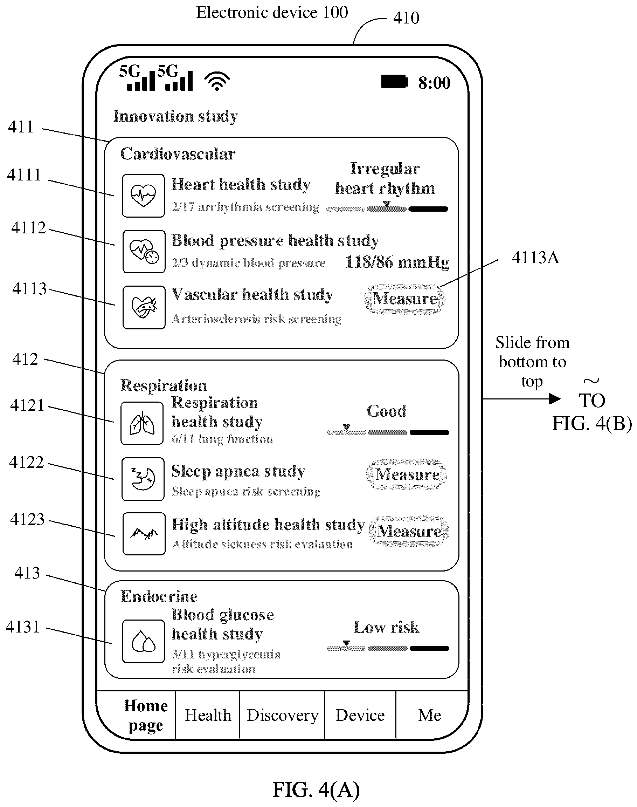

This application provides a display method, applied to an electronic device. The electronic device may display a first application card in a first interface. The first application card may include a second application card (for example, as shown in FIG. 4(A), a second application card "Respiration health study" may be displayed in a first application card "Respiration"), and a measurement result (which may also be referred to as result data) of a physiological parameter is not displayed in the second application card. After obtaining measurement data of at least one type of physiological parameter of a user, the electronic device may generate a new first application card based on the at least one type of measurement data. A measurement result of the physiological parameter is displayed in the new first application card, and the electronic device may display the new first application card on the top of a second interface. It may be understood that, that the new first application card in the second interface is displayed above the first application card, and the second application card is not displayed in the first application card may be understood as that after obtaining the measurement data, the electronic device switches the second application card to the new first application card for display, and display the result data of the physiological parameter in the new first application card. The user may directly obtain the measurement result of the at least one type of physiological parameter by using the new first application card displayed on the top of the second interface, and does not need to perform operations such as starting a plurality of apps, switching pages a plurality of times, and dragging and sliding a scroll bar on a page a plurality of times. Therefore, according to the foregoing method, the user can quickly and conveniently obtain the measurement result of the physiological parameter, operations that need to be performed by the user to obtain the measurement result are reduced, and user operations are greatly reduced especially when there are a large quantity of physiological parameters. This effectively improves the user's efficiency of obtaining information, and improves user experience.

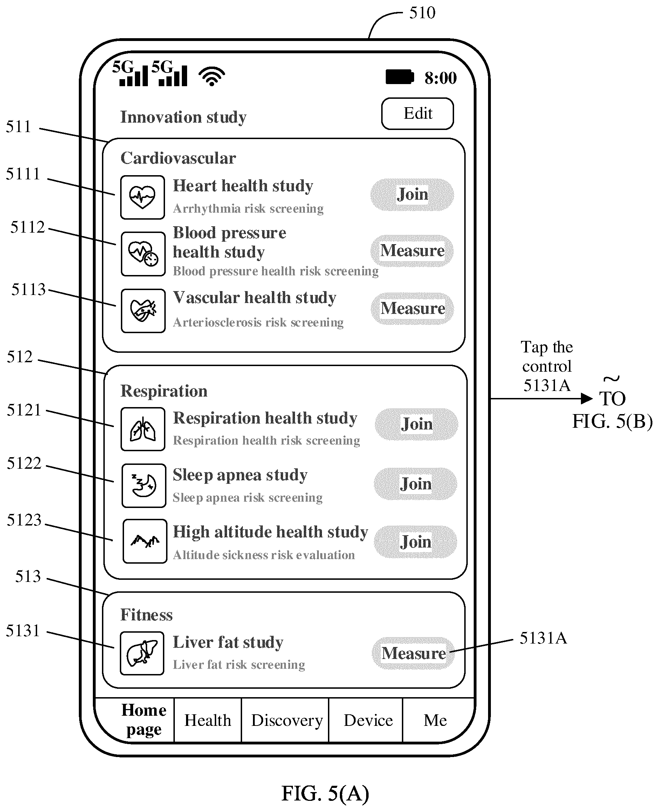

The first application card in the first interface may indicate a physiological category. For example, the first application card is a "Cardiovascular" application card, a "Respiration" application card, or a "Fitness" application card in FIG. 5(A). Each first application card may include at least one second application card. For example, the "Cardiovascular" application card in FIG. 5(A) includes three second application cards: a "Heart health study" application card, a "Blood pressure health study" application card, and a "Vascular health study" application card. It should be understood that there may be one or more second application cards displayed in the first application card. The second application card may indicate a measurement function of a physiological parameter. The second application card may be an application card that is displayed when the measurement function corresponding to the application card is not enabled/that does not include information such as a valid measurement result. The second application card may be used to trigger measurement of a corresponding physiological parameter. It may be understood that the electronic device provides, for the user by using the second application card, an entry of a measurement function of a corresponding physiological parameter. In an implementation, the electronic device may receive a user operation for the second application card, to trigger enabling of the measurement function of the physiological parameter corresponding to the second application card.

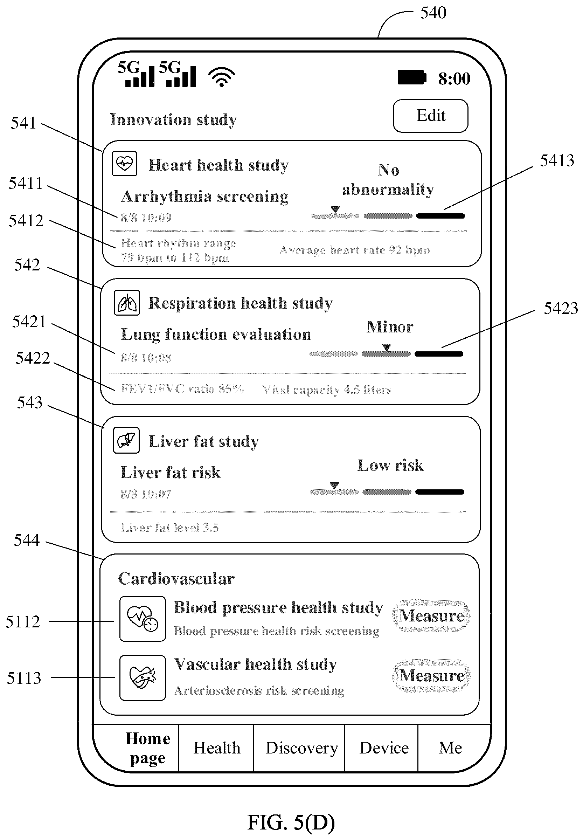

The new first application card may be used to display a measurement result of a physiological parameter, to provide the measurement result for the user to view. For example, the new first application card is the "Heart health study" application card, the "Respiration health study" application card, the "Liver fat study" application card in FIG. 5(D). In an implementation, the electronic device may receive a user operation for the new first application card, to display detailed content of the measurement result of the physiological parameter indicated by the new first application card. In an implementation, the electronic device may receive the user operation for the new first application card, to adjust a display location/display order of the new first application card. In an implementation, the electronic device may receive a user operation for a plurality of new first application cards, to fuse the plurality of application cards to obtain a fused application card. The fused application card may indicate a new physiological parameter, and is used to display a measurement result of the new physiological parameter.

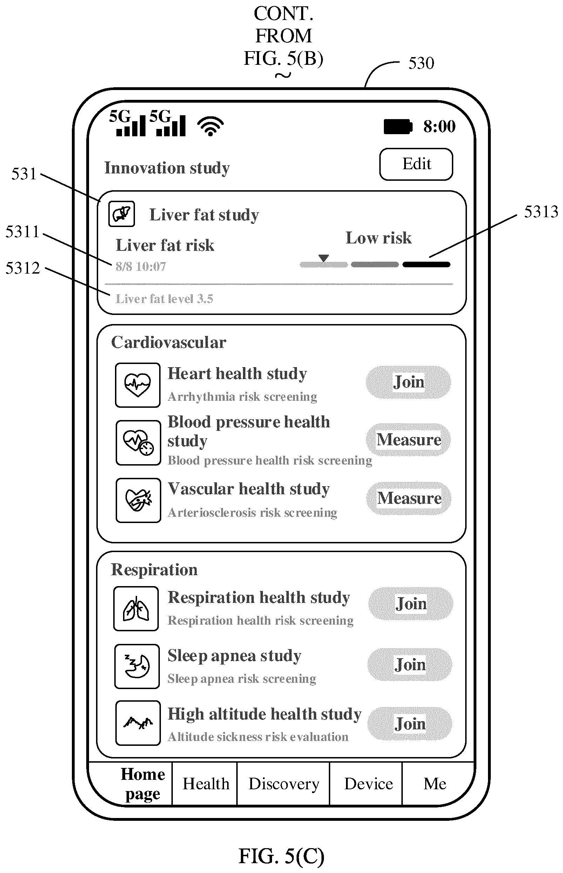

In some examples, the electronic device may display a first interface. The first interface is, for example, a user interface shown in FIG. 5(A). The first application card "Fitness" in the first interface includes the second application card "Liver fat study". After the electronic device obtains measurement data of liver fat of the user, as shown in FIG. 5(C), the electronic device switches the second application card "Liver fat study" to a new first application card "Liver fat study" for display. The new first application card "Liver fat study" includes a new second application card "Liver fat risk", and the new first application card "Liver fat study" is displayed above the first application card "Cardiovascular" and the first application card "Respiration".

The application card in this application may be a control in which content in an application is displayed in a form of card in a user interface of the application.

The card in this application may be a control displayed in a form of card in an interface other than a user interface of an application, for example, a home screen, or a leftmost screen of the electronic device.

The physiological parameter in this application includes, but is not limited to, for example, a heart rate, blood pressure, vascular sclerosis, a lung function, sleep apnea, altitude sickness, blood glucose, and liver fat. A physiological category to which the heart rate, the blood pressure, and the vascular sclerosis belong is cardiovascular, a physiological category to which the lung function, the sleep apnea, and the altitude sickness belong is respiration, a physiological category to which the blood glucose belongs is endocrine, and a physiological category to which the liver fat belongs is fitness.

In this application, the electronic device may be a mobile phone, a tablet computer, a handheld computer, a desktop computer, a laptop computer, an ultra-mobile personal computer (ultra-mobile personal computer, UMPC), a netbook, a cellular phone, a personal digital assistant (personal digital assistant, PDA), a smart home device like a smart television or a smart camera, a wearable device like a smart band, a smart watch, or smart glasses, an extended reality (extended reality, XR) device like an augmented reality (augmented reality, AR) device, a virtual reality (virtual reality, VR) device, or a mixed reality (mixed reality, MR) device, a vehicle-mounted device, or a smart city device. A specific type of the electronic device is not specially limited in embodiments of this application.

In an implementation, the electronic device may be provided with a foldable display (which may be referred to as a foldable screen). The electronic device may be referred to as a foldable electronic device. Folding the foldable screen may also be referred to as folding the electronic device. A physical status/posture of the foldable screen may also be referred to as a physical status of the electronic device.

The following describes a structure of an example electronic device provided in embodiments of this application.

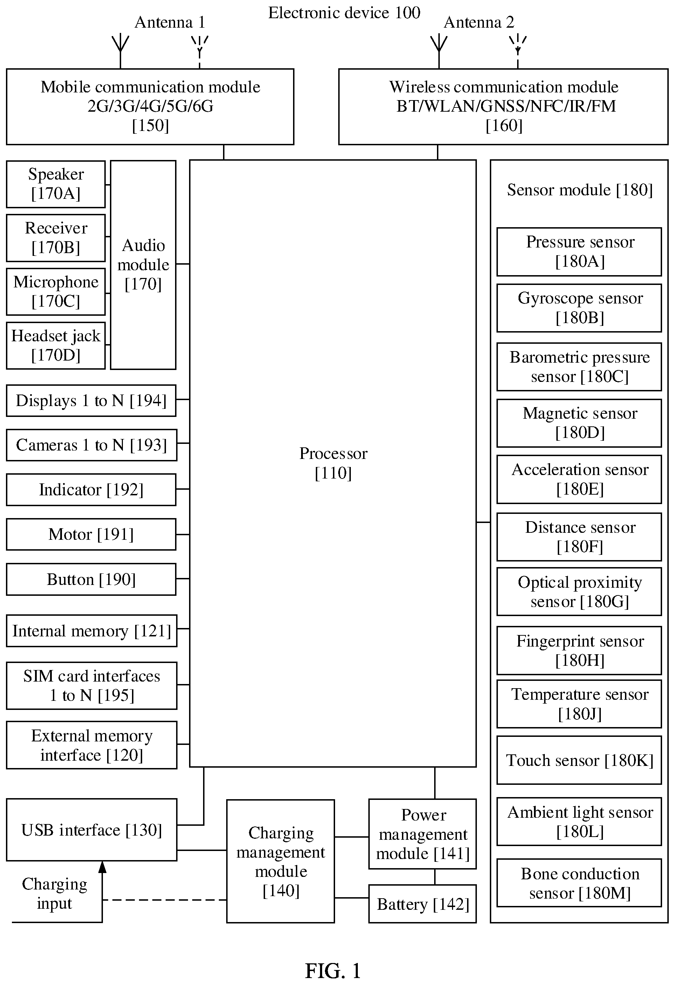

FIG. 1 is an example of a diagram of a hardware structure of an electronic device 100.

As shown in FIG. 1, the electronic device 100 may include a processor 110, an external memory interface 120, an internal memory 121, a universal serial bus (universal serial bus, USB) interface 130, a charging management module 140, a power management module 141, a battery 142, an antenna 1, an antenna 2, a mobile communication module 150, a wireless communication module 160, an audio module 170, a speaker 170A, a receiver 170B, a microphone 170C, a headset jack 170D, a sensor module 180, a button 190, a motor 191, an indicator 192, a camera 193, a display 194, a subscriber identity module (subscriber identity module, SIM) card interface 195, and the like. The sensor module 180 may include a pressure sensor 180A, a gyroscope sensor 180B, a barometric pressure sensor 180C, a magnetic sensor 180D, an acceleration sensor 180E, a distance sensor 180F, an optical proximity sensor 180G, a fingerprint sensor 180H, a temperature sensor 180J, a touch sensor 180K, an ambient light sensor 180L, a bone conduction sensor 180M, and the like.

It may be understood that the structure shown in this embodiment of the present invention does not constitute a specific limitation on the electronic device 100. In some other embodiments of this application, the electronic device 100 may include more or fewer components than those shown in the figure, or combine some components, or split some components, or have different component arrangements. The components shown in the figure may be implemented by hardware, software, or a combination of software and hardware.

The processor 110 may include one or more processing units. For example, the processor 110 may include an application processor (application processor, AP), a modem processor, a graphics processing unit (graphics processing unit, GPU), an image signal processor (image signal processor, ISP), a controller, a video codec, a digital signal processor (digital signal processor, DSP), a baseband processor, and/or a neural-network processing unit (neural-network processing unit, NPU). Different processing units may be independent components, or may be integrated into one or more processors.

The controller may generate an operation control signal based on instruction operation code and a time sequence signal, to control instruction fetching and instruction execution.

A memory may be further disposed in the processor 110, and is configured to store instructions and data. In an implementation, the memory in the processor 110 is a cache memory. The memory may store instructions or data that has been used or cyclically used by the processor 110. If the processor 110 needs to use the instructions or the data again, the processor may directly invoke the instructions or the data from the memory. This avoids repeated access, reduces waiting time of the processor 110, and improves system efficiency.

The charging management module 140 is configured to receive a charging input from the charger. The power management module 141 is configured to connect to the battery 142, the charging management module 140, and the processor 110. The power management module 141 receives an input from the battery 142 and/or the charging management module 140, and supplies power to the processor 110, the internal memory 121, the display 194, the camera 193, the wireless communication module 160, and the like. The power management module 141 may be further configured to monitor parameters such as a battery capacity, a battery cycle count, and a battery health status (electric leakage or impedance). In another implementation, the power management module 141 may alternatively be disposed in the processor 110. In another implementation, the power management module 141 and the charging management module 140 may alternatively be disposed in a same device.

A wireless communication function of the electronic device 100 may be implemented through the antenna 1, the antenna 2, the mobile communication module 150, the wireless communication module 160, the modem processor, the baseband processor, and the like.

The antenna 1 and the antenna 2 are configured to transmit and receive an electromagnetic wave signal. Each antenna in the electronic device 100 may be configured to cover one or more communication frequency bands. Different antennas may be further multiplexed, to improve antenna utilization. For example, the antenna 1 may be multiplexed as a diversity antenna of a wireless local area network. In another implementation, the antenna may be used in combination with a tuning switch.

The mobile communication module 150 may provide a wireless communication solution that is applied to the electronic device 100 and that includes a second generation (second generation, 2G) mobile communication technology/a third generation (third generation, 3G) mobile communication technology/a fourth generation (fourth generation, 4G) mobile communication technology/a fifth generation mobile (fifth generation, 5G) communication technology/a sixth generation (sixth generation, 6G) mobile communication technology. The mobile communication module 150 may include at least one filter, a switch, a power amplifier, a low noise amplifier (low noise amplifier, LNA), and the like. The mobile communication module 150 may receive an electromagnetic wave through the antenna 1, perform processing such as filtering or amplification on the received electromagnetic wave, and transmit the electromagnetic wave to the modem processor for demodulation. The mobile communication module 150 may further amplify a signal modulated by the modem processor, and convert the signal into an electromagnetic wave for radiation through the antenna 1. In an implementation, at least some function modules of the mobile communication module 150 may be disposed in the processor 110. In an implementation, at least some function modules of the mobile communication module 150 may be disposed in a same component as at least some modules of the processor 110.

The modem processor may include a modulator and a demodulator. The modulator is configured to modulate a to-be-sent low-frequency baseband signal into a medium-high frequency signal. The demodulator is configured to demodulate a received electromagnetic wave signal into a low-frequency baseband signal. Then, the demodulator transmits the low-frequency baseband signal obtained through demodulation to the baseband processor for processing. The low-frequency baseband signal is processed by the baseband processor and then transmitted to the application processor. The application processor outputs a sound signal by an audio device (which is not limited to the speaker 170A, the receiver 170B, or the like), or displays an image or a video by the display 194. In an implementation, the modem processor may be an independent component. In another implementation, the modem processor may be independent of the processor 110, and is disposed in a same component as the mobile communication module 150 or another function module.

The wireless communication module 160 may provide a wireless communication solution that is applied to the electronic device 100 and that includes a wireless local area network (wireless local area network, WLAN) (for example, a wireless fidelity (wireless fidelity, Wi-Fi) network), Bluetooth (bluetooth, BT), a global navigation satellite system (global navigation satellite system, GNSS), frequency modulation (frequency modulation, FM), a near field communication (near field communication, NFC) technology, an infrared (infrared, IR) technology, or the like. The wireless communication module 160 may be one or more components integrating at least one communication processing module. The wireless communication module 160 receives an electromagnetic wave through the antenna 2, performs frequency modulation and filtering processing on an electromagnetic wave signal, and sends a processed signal to the processor 110. The wireless communication module 160 may further receive a to-be-sent signal from the processor 110, perform frequency modulation and amplification on the signal, and convert the signal into an electromagnetic wave for radiation through the antenna 2.

In an implementation, in the electronic device 100, the antenna 1 and the mobile communication module 150 are coupled, and the antenna 2 and the wireless communication module 160 are coupled, so that the electronic device 100 can communicate with a network and another device by using a wireless communication technology. The wireless communication technology may include a global system for mobile communications (global system for mobile communications, GSM), a general packet radio service (general packet radio service, GPRS), code division multiple access (code division multiple access, CDMA), wideband code division multiple access (wideband code division multiple access, WCDMA), time-division code division multiple access (time-division code division multiple access, TD-SCDMA), long term evolution (long term evolution, LTE), BT, a GNSS, a WLAN, NFC, FM, an IR technology, and/or the like. The GNSS may include a global positioning system (global positioning system, GPS), a global navigation satellite system (global navigation satellite system, GLONASS), a BeiDou navigation satellite system (beidou navigation satellite system, BDS), a quasi-zenith satellite system (quasi-zenith satellite system, QZSS), and/or a satellite based augmentation system (satellite based augmentation system, SBAS).

The electronic device 100 may implement a display function through the GPU, the display 194, the application processor, and the like. The GPU is a microprocessor for image processing, and is connected to the display 194 and the application processor. The GPU is configured to: perform mathematical and geometric computation, and render an image. The processor 110 may include one or more GPUs, which execute program instructions to generate or change display information.

The display 194 is configured to display an image, a video, and the like. The display 194 includes a display panel. The display panel may be a liquid crystal display (liquid crystal display, LCD), an organic light-emitting diode (organic light-emitting diode, OLED), an active-matrix organic light emitting diode (active-matrix organic light emitting diode, AMOLED), a flexible light-emitting diode (flexible light-emitting diode, FLED), a mini-LED, a micro-LED, a micro-OLED, a quantum dot light emitting diode (quantum dot light emitting diode, QLED), or the like. In an implementation, the electronic device 100 may include one or N displays 194, where N is a positive integer greater than 1.

The electronic device 100 may implement a photographing function through the camera 193, the ISP, the video codec, the GPU, the display 194, the application processor and the like.

The ISP is configured to process data fed back by the camera 193. For example, during photographing, a shutter is pressed, and light is transmitted to a photosensitive element of the camera through a lens. An optical signal is converted into an electrical signal, and the photosensitive element of the camera transmits the electrical signal to the ISP for processing, to convert the electrical signal into a visible image. The ISP may further perform algorithm optimization on noise, brightness, and color of the image. The ISP may further optimize parameters such as exposure and a color temperature of a photographing scenario. In an implementation, the ISP may be disposed in the camera 193.

The camera 193 is configured to capture a static image or a video. An optical image of an object is generated through the lens, and is projected onto the photosensitive element. The photosensitive element may be a charge coupled device (charge coupled device, CCD) or a complementary metal-oxide-semiconductor (complementary metal-oxide-semiconductor, CMOS) phototransistor. The photosensitive element converts an optical signal into an electrical signal, and then transmits the electrical signal to the ISP to convert the electrical signal into a digital image signal. The ISP outputs the digital image signal to the DSP for processing. The DSP converts the digital image signal into an image signal in a standard format like RGB or YUV. In an implementation, the electronic device 100 may include one or N cameras 193, where N is a positive integer greater than 1.

The external memory interface 120 may be used to connect to an external storage card, for example, a micro SD card, to extend a storage capability of the electronic device 100. The external memory card communicates with the processor 110 through the external memory interface 120, to implement a data storage function. For example, files such as music and videos are stored in the external storage card.

The internal memory 121 may be configured to store computer-executable program code. The executable program code includes instructions. The internal memory 121 may include a program storage area and a data storage area. The program storage area may store an operating system, an application required by at least one function (for example, a voice playing function or an image playing function), and the like. The data storage area may store data (such as audio data and an address book) created during use of the electronic device 100, and the like. In addition, the internal memory 121 may include a high-speed random access memory, or may include a nonvolatile memory, for example, at least one magnetic disk storage device, a flash memory, or a universal flash storage (universal flash storage, UFS). The processor 110 runs instructions stored in the internal memory 121 and/or instructions stored in the memory disposed in the processor, to perform various function applications and data processing of the electronic device 100.

The electronic device 100 may implement an audio function by using the audio module 170, the speaker 170A, the receiver 170B, the microphone 170C, the headset jack 170D, the application processor.

The audio module 170 is configured to convert digital audio information into an analog audio signal for output, and is also configured to convert analog audio input into a digital audio signal. The audio module 170 may be further configured to encode and decode an audio signal.

The speaker 170A, also referred to as a "loudspeaker", is configured to convert an audio electrical signal into a sound signal.

The receiver 170B, also referred to as an "earpiece", is configured to convert an audio electrical signal into a sound signal.

The microphone 170C, also referred to as a "mike" or a "mic", is configured to convert a sound signal into an electrical signal.

The headset jack 170D is configured to connect to a wired headset.

The pressure sensor 180A is configured to sense a pressure signal, and can convert the pressure signal into an electrical signal. In an implementation, the pressure sensor 180A may be disposed on the display 194. There are a plurality of types of pressure sensors 180A, such as a resistive pressure sensor, an inductive pressure sensor, and a capacitive pressure sensor. The capacitive pressure sensor may include at least two parallel plates made of conductive materials. When a force is applied to the pressure sensor 180A, capacitance between electrodes changes. The electronic device 100 determines pressure intensity based on the change in the capacitance. When a touch operation is performed on the display 194, the electronic device 100 detects intensity of the touch operation through the pressure sensor 180A. The electronic device 100 may also calculate a touch location based on a detection signal of the pressure sensor 180A. In an implementation, touch operations that are performed at a same touch location but have different touch operation intensity may correspond to different operation instructions.

The gyroscope sensor 180B may be configured to determine a moving posture of the electronic device 100. In an implementation, an angular velocity of the electronic device 100 around three axes (namely, axes x, y, and z) may be determined by using the gyroscope sensor 180B.

The barometric pressure sensor 180C is configured to measure barometric pressure.

The magnetic sensor 180D includes a Hall sensor. The electronic device 100 may detect opening and closing of a flip cover by using the magnetic sensor 180D.

The acceleration sensor 180E may detect accelerations in various directions (usually on three axes) of the electronic device 100.

The distance sensor 180F is configured to measure a distance.

The optical proximity sensor 180G may include, for example, a light-emitting diode (LED) and an optical detector, for example, a photodiode. The light-emitting diode may be an infrared light-emitting diode. The electronic device 100 emits infrared light by using the light-emitting diode. The electronic device 100 detects infrared reflected light from a nearby object through the photodiode. When sufficient reflected light is detected, it may be determined that there is an object near the electronic device 100. When insufficient reflected light is detected, the electronic device 100 may determine that there is no object near the electronic device 100.

The ambient light sensor 180L is configured to sense ambient light brightness.

The fingerprint sensor 180H is configured to collect a fingerprint. The electronic device 100 may use a feature of the collected fingerprint to implement fingerprint-based unlocking, application lock access, fingerprint-based photographing, fingerprint-based call answering, and the like.

The temperature sensor 180J is configured to detect a temperature.

The touch sensor 180K is also referred to as a "touch component". The touch sensor 180K may be disposed on the display 194, and the touch sensor 180K and the display 194 form a touchscreen, which is also referred to as a "touch screen". The touch sensor 180K is configured to detect a touch operation performed on or near the touch sensor. The touch sensor may transfer the detected touch operation to the application processor to determine a type of a touch event. A visual output related to the touch operation may be provided through the display 194. In some other embodiments, the touch sensor 180K may alternatively be disposed on a surface of the electronic device 100 at a location different from that of the display 194.

The bone conduction sensor 180M may obtain a vibration signal.

The button 190 includes a power button, a volume button, and the like. The button 190 may be a mechanical button, or may be a touch button. The electronic device 100 may receive a button input, and generate a button signal input related to a user setting and function control of the electronic device 100.

The motor 191 may generate a vibration prompt. The indicator 192 may be an indicator light, and may be configured to indicate a charging status and a power change, or may be configured to indicate a message, a missed call, a notification, and the like. The SIM card interface 195 is configured to connect to a SIM card.

A software system of the electronic device 100 may use a layered architecture, an event-driven architecture, a microkernel architecture, a micro service architecture, or a cloud architecture. For example, a software system of the layered architecture may be an Android (Android) system, or may be a Harmony (harmony) operating system (operating system, OS), or another software system. In this embodiment of this application, an Android system with a layered architecture is used as an example to describe a software structure of the electronic device 100.

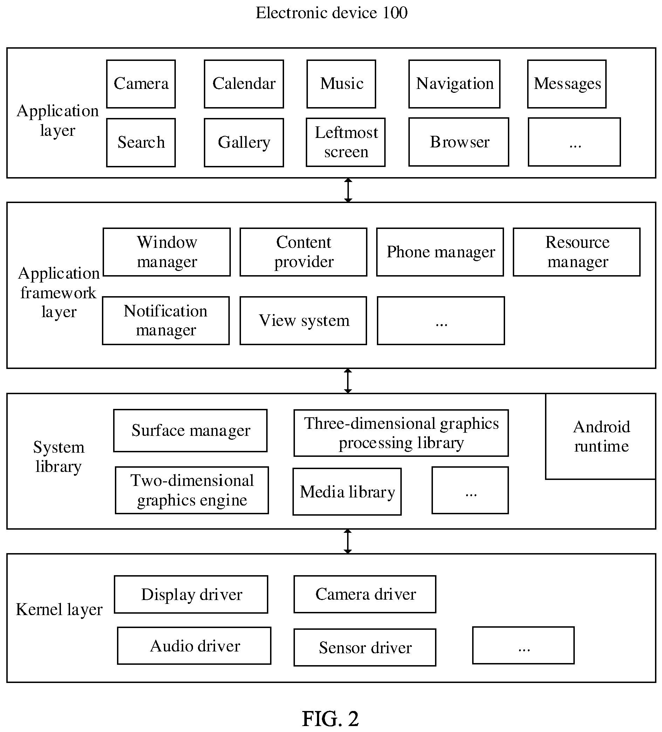

FIG. 2 shows an example of a diagram of a software architecture of the electronic device 100.

In a layered architecture, software is divided into several layers, and each layer has a clear role and task. The layers communicate with each other through a software interface. In an implementation, the Android system is divided into four layers: an application layer, an application framework layer, an Android runtime (Android runtime) and system library, and a kernel layer from top to bottom.

The application layer may include a series of application packages.

As shown in FIG. 2, the application layer may include applications such as Camera, Gallery, Music, Calendar, Messages, Phone, Navigation, Bluetooth, Browser, and Projection.

The application framework layer provides an application programming interface (application programming interface, API) and a programming framework for an application at the application layer. The application framework layer includes some predefined functions.

As shown in FIG. 2, the application framework layer may include a window manager, a content provider, a view system, a phone manager, a resource manager, a notification manager, and the like.

The window manager is configured to manage a window program. The window manager may obtain a size of a display, determine whether there is a status bar, perform screen locking, take a screenshot, and the like.

The content provider is configured to: store and obtain data, and enable the data to be accessed by an application. The data may include a video, an image, an audio, calls that are made and answered, a browsing history and a bookmark, an address book, and the like.

The view system includes visual controls such as a control for displaying a text and a control for displaying an image. The view system may be configured to construct an application. A display interface may include one or more views. For example, a display interface including an SMS message notification icon may include a text display view and an image display view.

The phone manager is configured to provide a communication function for the electronic device 100, for example, management of a call status (including answering, declining, or the like).

The resource manager provides various resources such as a localized character string, an icon, an image, a layout file, and a video file for an application.

The notification manager enables an application to display notification information in a status bar, and may be configured to convey a notification-type message that may automatically disappear after a short pause without requiring user interaction. For example, the notification manager is configured to notify download completion, give a message notification, and the like. The notification manager may alternatively be a notification that appears in a top status bar of the system in a form of graph or scroll bar text, for example, a notification of an application that is run on the background, or may be a notification that appears on a screen in a form of dialog window. For example, text information is displayed in the status bar, an alert tone is made, the electronic device vibrates, or the indicator light blinks.

The Android runtime includes a core library and a virtual machine. The Android runtime is responsible for scheduling and management of the Android system.

The core library includes two parts: a function that needs to be called in Java language and a core library of Android.

The application layer and the application framework layer run on the virtual machine. The virtual machine executes java files of the application layer and the application framework layer as binary files. The virtual machine is configured to implement functions such as object lifecycle management, stack management, thread management, security and exception management, and garbage collection.

The system library may include a plurality of functional modules, for example, a surface manager (surface manager), a media library (Media library), a three-dimensional graphics processing library (for example, OpenGL ES), and a 2D graphics engine (for example, SGL).

The surface manager is configured to manage a display subsystem and provide fusion of 2D and 3D layers for a plurality of applications.

The media library supports playback and recording in a plurality of commonly used audio and video formats, and static image files. The media library may support a plurality of audio and video coding formats, for example, MPEG-4, H.264, MP3, AAC, AMR, JPG, and PNG.

The three-dimensional graphics processing library is configured to implement three-dimensional graphics drawing, image rendering, composition, layer processing, and the like.

The 2D graphics engine is a drawing engine for 2D drawing.

The kernel layer is a layer between hardware and software. The kernel layer includes at least a display driver, a camera driver, an audio driver, and a sensor driver.

The following describes an example of a working procedure of software and hardware of the electronic device 100 with reference to a display scenario.

When the touch sensor 180K receives a touch operation, a corresponding hardware interrupt is sent to the kernel layer. The kernel layer processes the touch operation into an original input event (including information such as touch coordinates and a time stamp of the touch operation). The original input event is stored at the kernel layer. The application framework layer obtains the original input event from the kernel layer, and identifies a control corresponding to the input event. For example, the touch operation is a touch tap operation, and a control corresponding to the tap operation is a control of a health application. The health application invokes an interface of the application framework layer, to control the display driver by invoking the kernel layer, and display a main interface of the health application on the display 194.

In some embodiments, as shown in FIG. 4(A), a layout of cards in the electronic device on a first page is as follows:

A first application card may be designed based on a human body physiological system, and includes, for example, first application cards such as a "Cardiovascular" application card, a "Respiration" application card, or an "Endocrine" application card.

It should be understood that the first page may include a left screen (for example, a "Home page" page displayed in FIG. 4(A), and the user may tap a control "Home page" to quickly display the page), a right screen (for example, a "Me" page displayed in FIG. 4(A), and the user may tap a "Me" control to quickly display the page), and a home page that are in an application, a lock screen page of the electronic device, any page of a home screen of the electronic device, a notification bar of the electronic device, and the like.

Each first application card may include one or more second application cards. For example, the first application card "Cardiovascular" includes three second application cards: "Heart health study", "Blood pressure health study", and "Vascular health study".

In some embodiments, the first application card and the second application card are in an inclusion relationship. When the user moves a location of the first application card, a location of the second application card included in the first application card is moved. For example, the first application card "Cardiovascular" in FIG. 4(A) is moved to the bottom of the first page. Correspondingly, the second application cards "Heart health study", "Blood pressure health study", and "Vascular health study" included in the first application card "Cardiovascular" are also moved to the bottom of the first page, but orders of the second application cards "Heart health study", "Blood pressure health study", and "Vascular health study" in the first application card "Cardiovascular" remain unchanged.

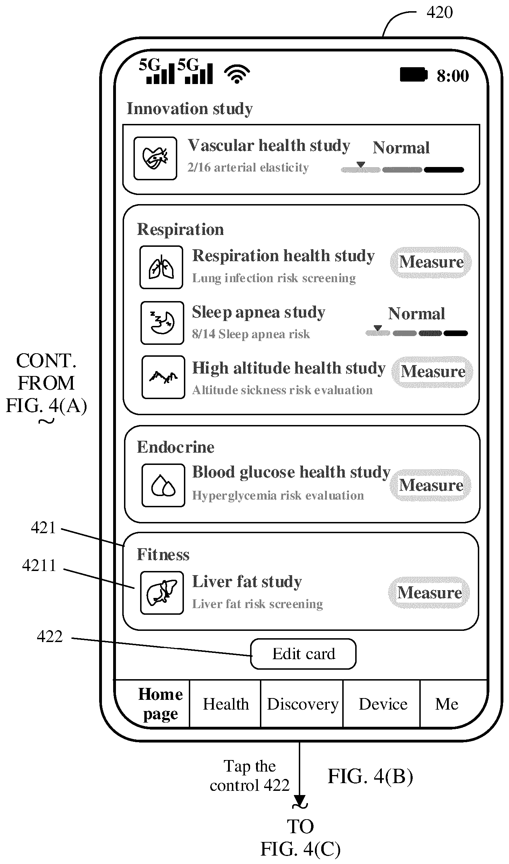

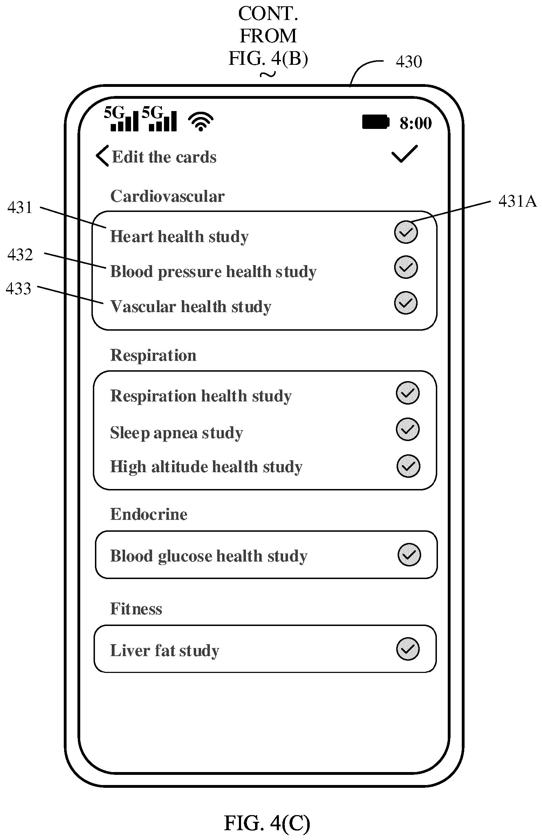

In some embodiments, the second application card may have at least three statuses. A first state of the second application card is a hidden state, that is, the electronic device does not display, on the first page, the second application card in the hidden state, but the second application card may be manually edited and added by the user. For example, the user may tap an "Edit card" control on a user interface shown in FIG. 4(B), and the electronic device displays an interface shown in FIG. 4(C). The user may choose to add a corresponding second application card and/or first application card to the first page of the electronic device for display. A second state of the second application card is an initial state (for example, a measurement result unavailable state), and no detection result is displayed in the second application card. For example, in the second application card "Respiration health study" shown in FIG. 5(A), no measurement result is currently displayed in the second application card. In some embodiments, a second application card in which there is no "user measurement result" may display a control "Join", to prompt the user to measure a physiological parameter. A third state is a measurement result available state. For example, the second application card "Heart health study", the second application card "Blood pressure health study", the second application card "Blood glucose health study", or the second application card "Respiration health study" shown in FIG. 4(A). When the user obtains measurement results after measuring physiological parameters by using the electronic device, the electronic device displays the measurement results in the second application cards. In some embodiments, different color areas may be marked based on the measurement results, and different colors are used to identify intervals in which the measurement results are located. For example, as shown in FIG. 4(A), for the second application card "Blood glucose health study", a measurement result indicates a low risk, and therefore a first color segment in the card is marked.

The first application card and the second application card are bound together, so that the user can operate and manage the cards conveniently. The user can move or change cards of different sub-categories under a same category at one time.

When the second application card changes from the second state to the third state, the display location of the second application card on the first page remains unchanged. An embodiment of this application further provides a dynamic card display method, to make card differentiated, to conveniently prompt a user which card has a measurement result, and to facilitate interaction between the user and the card.

The dynamic card display method provided in this application includes: After a user performs an operation, a status of a second application card changes, a display location is removed from an original first application card, and the second application card is independently displayed at another location. Optionally, notifications such as a pop-up window, a scrolling message, voice, and a color change may be given. After the user performs a type of operation like deletion/exit, the second application card in a second state is no longer independently displayed in the another location, and is moved back to the original first application card. Optionally, notifications such as a pop-up window, a scrolling message, voice, and a color change may be given.

The display method is applicable to terminal display devices such as a mobile phone, a watch, a television, a health check device, and a vehicle-mounted terminal.

As shown in FIG. 5(A) to FIG. 5(D), each first application card may include one or more second application cards in the second state. For example, the first application card "Cardiovascular" includes three second application cards: "Heart health study", "Blood pressure health study", and "Vascular health study". The first application card that includes the second application card in the second state is displayed on the first page.

In an implementation, after the second application card is removed from the first application card, the first application card obtained after the second application card is removed is still referred to as the first application card. For example, the first application card "Cardiovascular" includes three second application cards: "Heart health study", "Blood pressure health study", and "Vascular health study". After the "Heart health study" is removed from the "Cardiovascular", a card including "Blood pressure health study" and "Vascular health study" is still referred to as the "Cardiovascular" card.

After the user performs an operation like measurement/monitoring/recording, a measurement result is displayed in at least one second application card in the first application card, and the status of the second application card changes from the second state to the third state. In some embodiments, a display manner of the second application card changes.

A possible display method includes: The electronic device removes the second application card from the original first application card to become a new first application card, and displays the new first application card and the original first application card on a same page. Optionally, a priority of an arrangement order of the second application card after the location is changed is higher than that of the original first application card and another first application card that includes only the second application card in the second state. For example, as shown in FIG. 5(A), the first application card "Respiration" includes the second application card "Respiration health study". As shown in FIG. 5(D), after the electronic device detects that the user starts measurement on the "Respiration health study" and obtains a result, the electronic device changes the second application card "Respiration health study" into a new first application card "Respiration health study". The new first application card "Respiration health study" includes a new second application card "Lung function evaluation". In the new first application card "Respiration health study", a measurement result is "Minor". In some embodiments, new first application cards may be sorted from top to bottom based on measurement time of the user. For example, as shown in FIG. 5(D), the user sequentially performs "Arrhythmia screening", "Lung function evaluation", and "Liver fat risk". In this case, the electronic device may sort new first application cards from top to bottom based on measurement time or obtaining time of measurement results. In some embodiments, the electronic device may also notify the new first application card in a manner like a scrolling message, voice, or a color change. In some embodiments, the electronic device may alternatively display the new first application card in a pop-up window (for example, in a message pop-up window manner), or display the new first application card on a home screen, to remind the user of the measurement result.

Another possible display method includes: The electronic device displays the first application card corresponding to the second application card on the top of a first page. For example, a second application card "High altitude health study" in the first application card "Respiration" has a measurement result, and the electronic device displays the first application card "Respiration" on the top. Optionally, the electronic device further displays the second application card on the top of the first application card. For example, the second application card "High altitude health study" in the first application card "Respiration" has the measurement result, and the electronic device displays the second application card "High altitude health study" on the top of the first application card "Respiration".

In some embodiments, the user may perform a type of operation like deletion/exit on the new first application card. After detecting that the user may perform the type of operation like the deletion/exit on the new first application card, the electronic device moves the new first application card back to the original first application card.

Implementations of the present invention may be combined at random to implement different technical effect.

The following describes a physical status of the foldable electronic device by using an example.

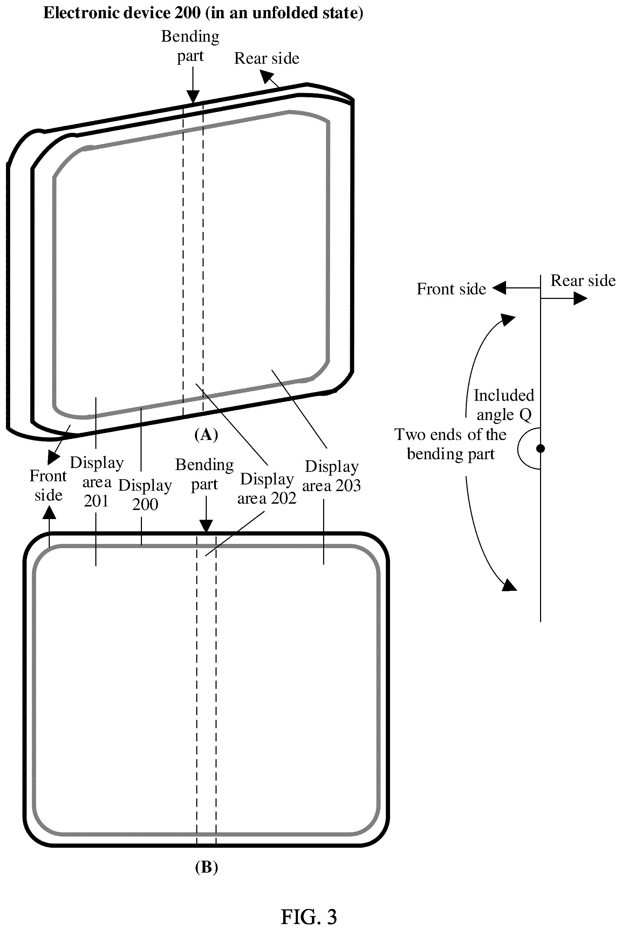

FIG. 3 is an example of a diagram of an electronic device 200 in an unfolded state. (A) and (B) in FIG. 3 separately are diagrams of the unfolded state from two view angles.

As shown in FIG. 3, the electronic device 200 may include a display 200. The display 200 is located on a front side of the electronic device 200. The electronic device 200 may include a bending part, and the bending part may be bent to the front side. For example, a value range of an included angle Q between two ends of the bending part shown in FIG. 3 is [0, 180 degrees]. This is not limited thereto. In some other examples, the bending part may also be bent to a rear side. For example, the value range of the included angle Q between the two ends of the bending part shown in FIG. 3 is [180 degrees, 360 degrees].

As shown in FIG. 3, the electronic device 200 may be in an unfolded state, and the included angle Q between the two ends of the bending part of the electronic device 200 is approximately 180 degrees. In this case, the display 200 is in an unfolded state, and the bending angle Q of the display 200 is approximately 180 degrees. This is not limited thereto. In another implementation, the included angle Q may be greater than or equal to 170 degrees and less than or equal to 180 degrees. A specific value of the included angle Q between the two ends of the bending part of the electronic device 200 in the unfolded state is not limited in this application.

As shown in (A) and (B) in FIG. 3, the display 200 may include a display area 201, a display area 202, and a display area 203. The display area 202 may be bent, and is located on the bending part of the electronic device 200. The two ends of the bending part of the electronic device 200 are respectively connected to the display area 201 and the display area 203. The included angle Q (also the bending angle Q of the display 200) between the two ends of the bending part of the electronic device 200 may also be understood as an included angle between a plane on which the display area 201 is located and a plane on which the display area 203 is located. When the display 200 is in the unfolded state, the display area 201 and the display area 203 are equivalent to being on a same plane.

In an implementation, the display 200 may be a flexible display of the electronic device 200. Optionally, the display area 201, the display area 202, and the display area 203 are different areas on the flexible display, and are all configured to display a user interface. In another implementation, the display 200 may be a display formed by splicing a rigid display, a flexible display, and a connection component like a chain. In some examples, the display 200 may be formed by splicing two rigid displays and one flexible display. The display area 201 and the display area 203 may be respectively areas on the two rigid displays, the display area 202 may be an area on the one flexible display, and are all configured to display the user interface. This is not limited thereto. In some other examples, the display 200 may be formed by splicing two rigid displays and a chain configured to connect the two rigid displays. The display area 201 and the display area 203 may be respectively areas on the two rigid displays, and both are configured to display the user interface. The display area 202 may be the foregoing chain configured to connect the two rigid displays.

The following describes application scenarios in embodiments of this application and diagrams of user interfaces in the scenarios.

FIG. 4(A) to FIG. 4(C) are a diagram of an example of a user interface.

As shown in FIG. 4(A), an electronic device 100 may display a user interface 410 (including a title "Innovation study"), and the user interface 410 is a user interface of a health application. The user interface 410 may include a plurality of first application cards, any first application card may indicate one type of physiological category, and any first application card may include at least one application card that indicates a physiological parameter of a corresponding physiological category. For example, a first application card 411 (including a title "Cardiovascular") may indicate a cardiovascular physiological type, a first application card 412 (including a title "Respiration") may indicate a respiration physiological type, and a first application card 413 (including a title "Endocrine") may indicate an endocrine physiological type. Any first application card may include at least one second application card. For example, the first application card 411 may include a second application card 4111, a second application card 4112, and a second application card 4113, the first application card 412 may include a second application card 4121, a second application card 4122, and a second application card 4123, and the first application card 413 may include a second application card 4131. A second state of the second application card is an initial state, and may indicate a measurement function of a type of physiological parameter. For example, the second application card 4113 includes characters "Vascular health study" and "Arteriosclerosis risk screening", and may indicate measurement functions of vascular sclerosis. The second application card 4122 includes characters "Sleep apnea study" and "Sleep apnea risk screening", and may indicate measurement functions of sleep apnea. The second application card 4123 includes characters "High altitude health study" and "Altitude sickness risk evaluation", and may indicate measurement functions of altitude sickness. The second application card in the second state may include a measurement control, and the measurement control may be used to trigger enabling of the measurement function corresponding to the application card. For example, the second application card 4113 may include a control 4113A, and the control 4113A may be used to trigger enabling of the measurement function of the vascular sclerosis. A third state of the second application card is a measurement result available state, and may be used to display result data of a corresponding physiological parameter. For example, the second application card 4111 includes characters "Heart health study", "2/17 arrhythmia screening", and "Irregular heart rhythm", and may indicate a measurement result of a heart rate: irregular heart rhythms occur during arrhythmia screening on February 17. Three color segments are displayed below the characters "Irregular heart rhythm", and a mark on a middle color segment may represent that the measurement result of the heart rate indicates irregular heart rhythms. The second application card 4121 includes characters "Respiration health study" and "Lung infection risk screening", and may indicate measurement functions of a lung function. The second application card 4112 includes characters "Blood pressure health study", "2/3 dynamic blood pressure", and "118/86 mmHg", and may indicate a measurement result of blood pressure: dynamic blood pressure on February 3 is 118/86 mmHg. The second application card 4121 includes characters "Respiration health study", "6/11 lung function", and "good", and may indicate a measurement result of a lung function: the lung function is good on June 11. Three color segments are displayed below the character "good", and a mark on a first color segment on the left may represent that the measurement result of the lung function is good. The second application card 4131 includes characters "Blood glucose health study", "3/11 hyperglycemia risk evaluation", and "Low risk", and may indicate a measurement result of blood glucose: blood glucose is at a low risk on March 11. Three color segments are displayed below the characters "Low risk", and a mark on a first color segment on the left may represent that the measurement result of the blood glucose is the low risk. In an implementation, the electronic device 100 may display, in response to a user operation (for example, the user operation is a sliding operation from bottom to top) for the user interface 410, a user interface 420 shown in FIG. 4(B).

As shown in FIG. 4(B), the user interface 420 is similar to the user interface 410, and a difference lies in that the user interface 420 further includes a first application card 421 (including a title "Fitness") and a control 422. The first application card 421 may indicate a fitness physiological type, and includes a second application card 4211. The second application card 4211 includes characters "Liver fat study" and "Liver fat risk screening", and may indicate a measurement function of liver fat. Characters "Edit card" are displayed in the control 422, which may be used to choose to display/hide at least one first application card and/or at least one second application card. In an implementation, the electronic device 100 may display, in response to a user operation (for example, the user operation is a touch operation) for the control 422, a user interface 430 shown in FIG. 4(C).

As shown in FIG. 4(C), the user interface 430 may be used to implement an editing function of an application card. The user interface 430 may include a plurality of titles, for example, "Cardiovascular", "Respiration", "Endocrine", and "Fitness". Any title may indicate one first application card. For example, the title "Cardiovascular" may indicate the first application card 411 in the user interface 410. At least one piece of text content is displayed below any title, and any piece of text content may indicate one second application card. For example, text content 431, text content 432, and text content 433 are displayed below the title "Cardiovascular". The text content 431 includes the characters "Heart health study", and may indicate the second application card 4111 in the user interface 410. The text content 432 includes the characters "Blood pressure health study", and may indicate the first application card 4112 in the user interface 410. The text content 433 includes the characters "Vascular health study", and may indicate the second application card 4113 in the user interface 410. A selection control is further displayed on the right of any piece of text content. For example, a selection control 431A is displayed on the right of the text content 431, and the selection control 431A may be used to select to display a corresponding second application card 4111 or cancel the selection.

Currently, the first application card in the health application includes both the second application card in the second state and the second application card in the third state. In other words, the second application cards in the two states are arranged in a mixed manner in the first application card to which the second application cards belong. For a specific example, refer to the user interface 410 shown in FIG. 4(A). If the user wants to view a measurement result of a physiological parameter (it is assumed that the measurement result is a measurement result of the liver fat), the user first searches the user interface 410 for an application card corresponding to the liver fat. When the application card is not found in the current user interface 410, the user performs a sliding operation to view other content. For example, the electronic device may display, in response to the sliding operation for the user interface 410, the user interface 420 shown in FIG. 4(B). In this case, the user may find the second application card 4211 in the user interface 420, that is, the application card corresponding to the liver fat, and the user may obtain information, for example, the measurement result of the liver fat by using the application card. The series of user operations are too cumbersome and time-consuming, especially when there are a large quantity of application cards. In addition, arrangement manners of the application cards in the user interface 410 and the user interface 420 make visual differentiation between the second application card in the second state and the second application card in the third state low for the user, and it is inconvenient for the user to browse and view information, for example, a measurement result that the user wants to view. Consequently, obtaining information is inefficient, and user experience is poor.

FIG. 5(A) to FIG. 5(D) are a diagram of an example of a user interface.

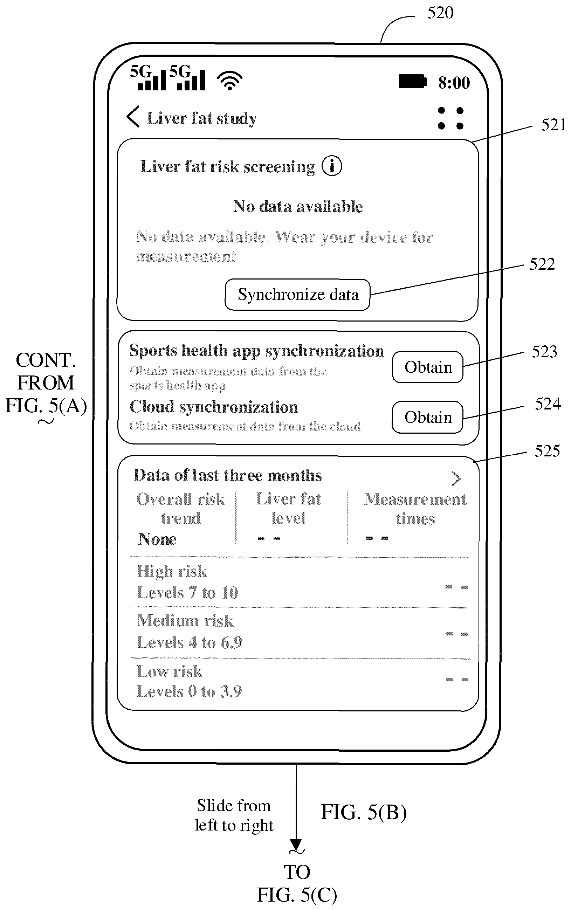

As shown in FIG. 5(A), the electronic device 100 may display a user interface 510. The user interface 510 is similar to the user interface 410 shown in FIG. 4(A), and a difference lies in that any first application card in the user interface 510 includes only at least one second application card in a second state. For example, a first application card 511 may include a second application card 5111, a second application card 5112, and a second application card 5113. A first application card 512 may include a second application card 5121, a second application card 5122, and a second application card 5123. A first application card 513 may include a second application card 5131. Measurement results are not displayed in these second application cards. In an implementation, the electronic device 100 may trigger, in response to a user operation (for example, the user operation is a touch operation) for a control 5131A in the second application card 5131, enabling of a measurement function of liver fat corresponding to the second application card 5131. In this case, the electronic device 100 may display a detailed interface of the application card. For a specific example, refer to a user interface 520 shown in FIG. 5(B).

As shown in FIG. 5(B), the user interface 520 may include an application card 521, a control 522, a control 523, a control 524, and an application card 525. The application card 521 in the user interface 520 is a card used when the measurement function of the liver fat is enabled for a first time and no measurement data of the liver fat is uploaded. Characters "Synchronize data" are displayed in the control 522, which may be used to obtain the measurement data of the liver fat. Specifically, the measurement data is obtained from a wearable device connected to the electronic device 100. A character "Obtain" is displayed in the control 523, which may be used to obtain the measurement data of the liver fat from an application related to the liver fat. Specifically, the measurement data is obtained from a sports health app. The character "Obtain" is displayed in the control 524, which may be used to obtain the measurement data of the liver fat from a cloud. The application card 525 may be used to display result data of the liver fat in a preset time period, for example, but is not limited to an overall risk trend, a liver fat level, and a quantity of measurement times. In an implementation, the electronic device 100 may display a selection interface of at least one wearable device in response to a user operation (for example, the user operation is a touch operation) for the control 522. The at least one wearable device may include but is not limited to a device discovered and/or connected by the electronic device 100 in a near field and/or far field communication manner, a device historically connected to the electronic device 100, a device recommended based on a corresponding physiological parameter, and the like. The electronic device 100 may receive a user operation (for example, the user operation is a touch operation) for one of the at least one wearable device, obtain the measurement data of the liver fat of the user in the device in response to the user operation, analyze the measurement data of the liver fat, generate result data of the liver fat, and display the result data in the application card 521 and the application card 525.

In an implementation, the electronic device 100 may exit a detailed interface of the liver fat in response to a user operation (for example, the user operation is a sliding operation from left to right from a left edge) for the user interface 520. In this case, the electronic device 100 may cancel displaying the second application card 5131 (the card indicating the measurement function of the liver fat) in the user interface 510 shown in FIG. 5(A), and display a generated new first application card. For a specific example, refer to a user interface 530 shown in FIG. 5(C).

As shown in FIG. 5(C), the user interface 530 is similar to the user interface 510 shown in FIG. 5(A), and a difference lies in that the user interface 530 does not include the second application card 5131 in the user interface 510, but includes a new first application card 531, and the new first application card 531 is displayed above the first application card 511 and the first application card 512. Optionally, because the first application card 513 includes only one second application card 5131, the user interface 530 does not include the first application card 513. The new first application card 531 may indicate the result data of the liver fat. The first application card 531 may include a new second application card (including a title "Liver fat risk"). The new second application card may include text content 5311, text content 5312, and a control 5313. The text content 5311 includes characters "8/8 10:07", which may indicate that generation time of the result data of the liver fat/the new first application card 531 is 10:07 on August 8. The text content 5312 includes characters "Liver fat level 3.5", the control 5313 includes three color segments, and a mark on a first color segment may represent that a risk level indicated by a measurement result of the liver fat is a low risk.

In an implementation, after the implementation shown in FIG. 5(A), the electronic device 100 may obtain the measurement data of the liver fat of the user, and generate the new first application card. The electronic device 100 may cancel displaying the second application card 5131 (the card indicating the measurement function of the liver fat) in the user interface 510 shown in FIG. 5(A), and display the generated new first application card. The electronic device 100 may further obtain measurement data of a lung function of the user. A specific process is similar to the foregoing process. The electronic device 100 may further obtain measurement data of a heart rate of the user. A specific process is similar to the foregoing process. In this case, the electronic device 100 may display a user interface 540 shown in FIG. 5(D).

As shown in FIG. 5(D), the user interface 540 is similar to the user interface 510 shown in FIG. 5(A), and a difference lies in that the user interface 540 does not include the second application card 5131, the second application card 5121, and the second application card 5111 in the user interface 510, but includes a new first application card 543, a new first application card 542, and a new first application card 541. The three new first application cards are displayed above a first application card 544, and the first application card 544 does not include the second application card 5111. The three new first application cards may respectively indicate result data of the foregoing three physiological parameters. The new first application card 541 may indicate result data of the heart rate. The new first application card 541 may include text content 5411, text content 5412, and a control 5413. The text content 5411 includes characters "8/8 10:09", which may indicate that generation time of the result data of the heart rate/the new first application card 541 is 10:09 on August 8. The text content 5412 includes characters "Heart rhythm range 79 bpm to 112 bpm" and "Average heart rate 92 bpm". The control 5413 includes three color segments and a mark on a first color segment on the left. Characters "No abnormality" are displayed above the control 5413, and may indicate that a risk level indicated by a measurement result of the heart rate is normal. The new first application card 542 may indicate result data of the lung function. The new first application card 542 may include text content 5421, text content 5422, and a control 5423. The text content 5421 includes characters "8/8 10:08", which may indicate that generation time of the result data of the lung function/the new first application card 542 is 10:08 on August 8. The text content 5422 includes characters "FEV1/FVC ratio 85%" and "Vital capacity 4.5 liters". The control 5423 includes three color segments, and a mark on a second color segment in the middle may represent that a risk level indicated by a measurement result of the lung function is minor. The new first application card 543 is similar to the new first application card 531 in the user interface 530 shown in FIG. 5(C). Details are not described again.

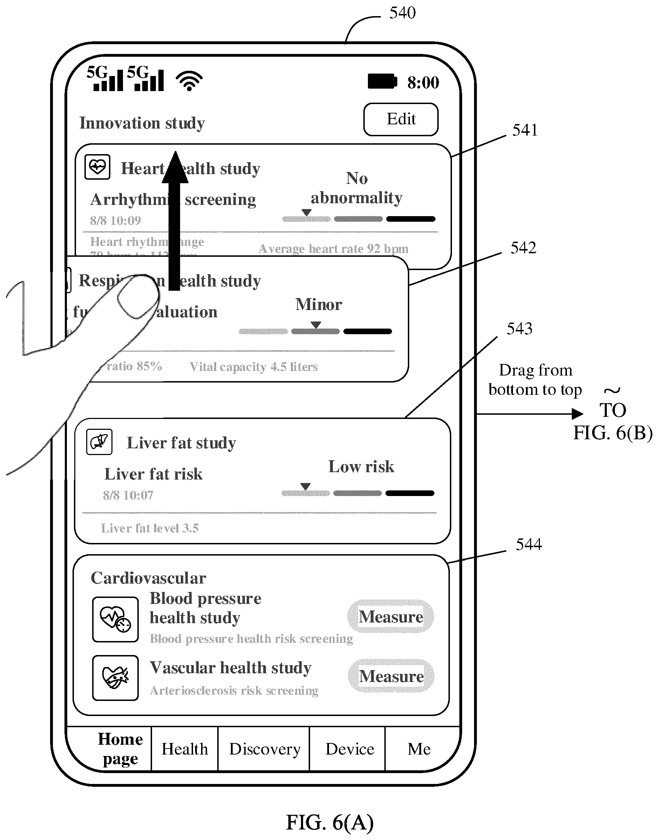

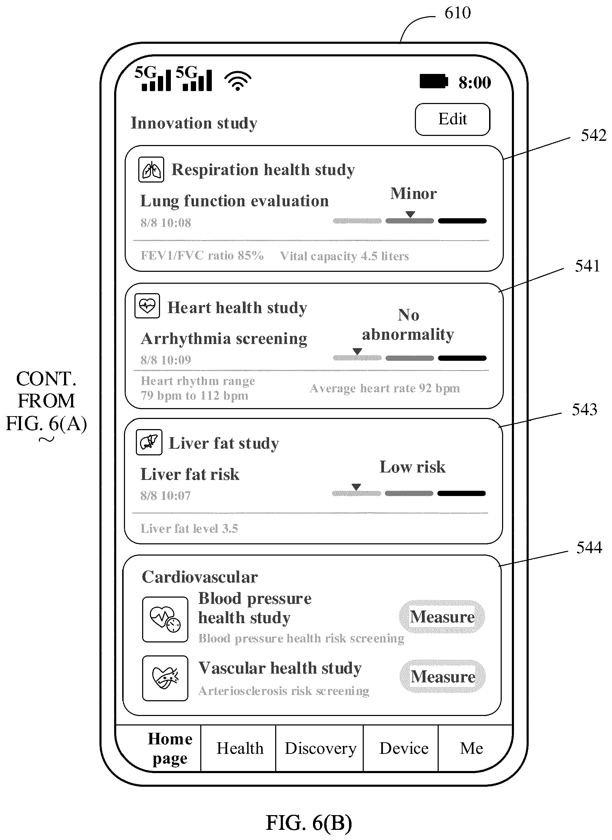

In an implementation, after the implementation shown in FIG. 5(D), the electronic device 100 may receive a user operation for the new first application card, and adjust a display location/display order of the new first application card in response to the user operation. In some examples, the electronic device 100 may receive a user operation (for example, the user operation is a drag operation from bottom to top) for the new first application card 542 in the user interface 540 shown in FIG. 6(A), and move a display location of the new first application card 542 to a location above the new first application card 541 in response to the user operation. In this case, the electronic device 100 may display a user interface 610 shown in FIG. 6(B).

As shown in FIG. 6(B), the user interface 610 is similar to the user interface 540, and a difference lies in that a display location of the new first application card 542 in the user interface 610 is above the new first application card 541. It may be understood that the electronic device 100 adjusts a top-bottom display order of application cards in the user interface 540: the new first application card 541, the new first application card 542, the new first application card 543, and the first application card 544 to a top-bottom display order in the user interface 610: the new first application card 542, the new first application card 541, the new first application card 543, and the first application card 544.

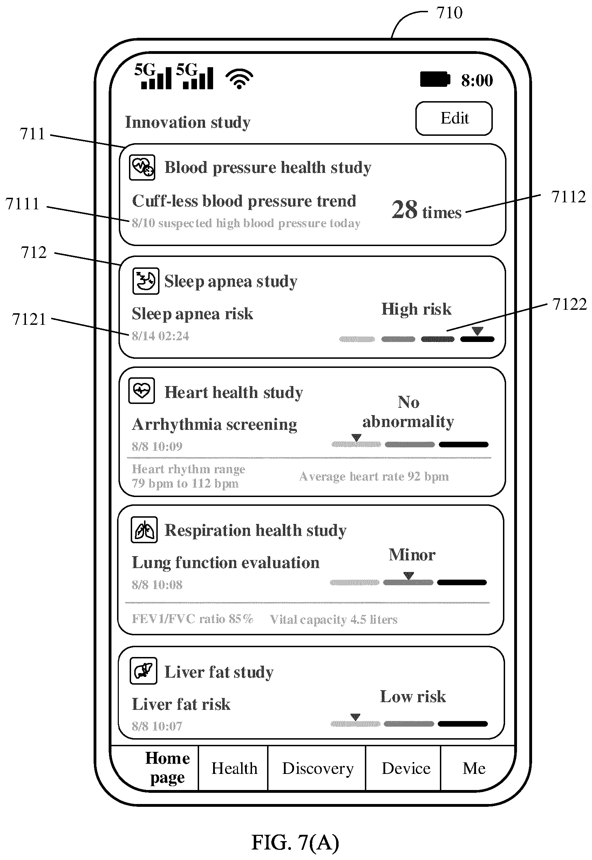

In an implementation, the electronic device 100 may fuse a plurality of new first application cards in response to a user operation for the plurality of new first application cards, and display a fused application card. For a specific example, refer to FIG. 7(A) to FIG. 7(D).

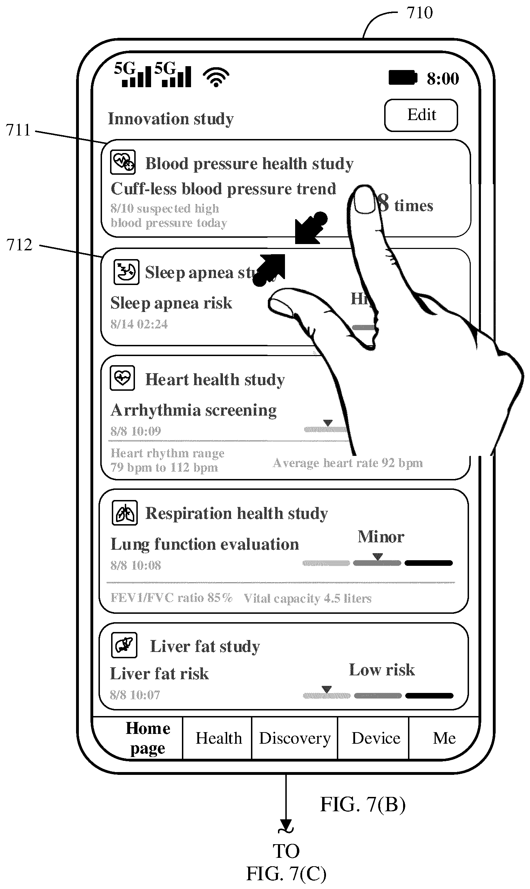

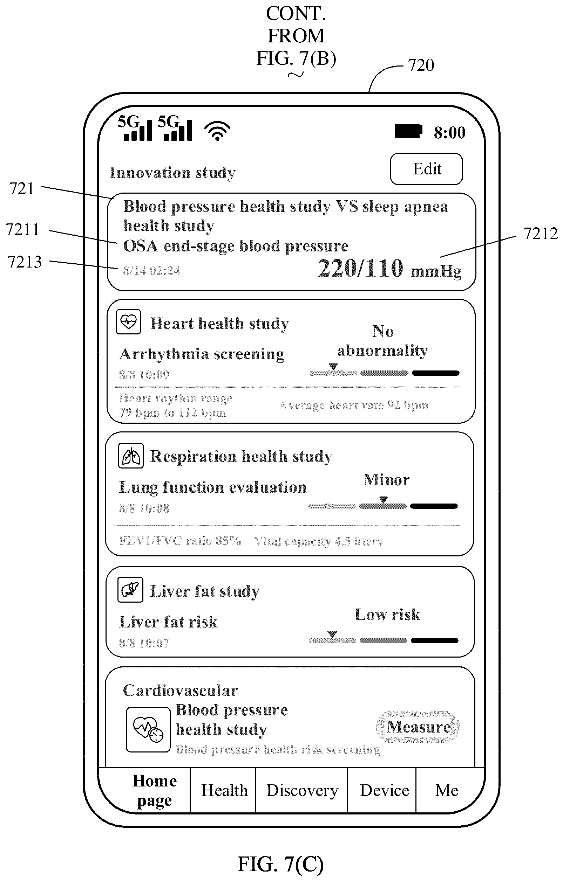

As shown in FIG. 7(A), the electronic device 100 may display a user interface 710. The user interface 710 may include a plurality of new first application cards, for example, a new first application card 711, a new first application card 712, and another new first application card. The new first application card 711 may indicate result data of blood pressure. The new first application card 711 may include text content 7111 and text content 7112. The text content 7111 includes characters "8/10 suspected high blood pressure today". The characters "8/10" may indicate that generation time of the result data of the blood pressure/the new first application card 711 is August 10. The characters "suspected high blood pressure today" and characters "28 times" included in the text content 7112 may represent that a measurement result of the blood pressure is that there are 28 times of suspected high blood pressure today. The new first application card 712 may indicate result data of sleep apnea. The new first application card 712 may include text content 7121 and a control 7122. The text content 7121 includes characters "8/14 02:24", which may represent that generation time of the result data of the sleep apnea/the new first application card 712 is 02:24 on August 14. The control 7122 includes four color segments and a mark on a rightmost color segment. Characters "High risk" are displayed above the control 7122, and may indicate that a risk level indicated by a measurement result of the sleep apnea is the high risk. As shown in FIG. 7(B), the electronic device 100 may receive a user operation (the user operation is, for example, a touch operation, and the touch operation is, for example, a pinch operation) for the new first application card 711 and the new first application card 712 in the user interface 710. In response to the user operation, the electronic device 100 may perform data fusion on measurement data of the blood pressure and measurement data of the sleep apnea, to obtain fused result data, fuse the new first application card 711 and the new first application card 712, and display the fused result data in a fused application card. In this case, the electronic device 100 may display a user interface 720 shown in FIG. 7(C).

As shown in FIG. 7(C), the user interface 720 is similar to the user interface 710, and a difference lies in that the user interface 720 may include a new first application card 721, but does not include the new first application card 711 or the new first application card 712. The new first application card 721 may be an application card obtained through fusion of the new first application card 711 and the new first application card 712. The new first application card 721 may indicate result data obtained through fusion of the blood pressure and the sleep apnea. The new first application card 721 may include text content 7211, text content 7212, and text content 7213. The text content 7211 includes characters "OSA end-stage blood pressure", which may represent a fused physiological parameter. The physiological parameter is related to the blood pressure and the sleep apnea. The text content 7212 includes "220/110 mmHg", which may represent that the fused result data is that the OSA end-stage blood pressure is 220/110 mmHg. The text content 7213 includes "8/14 02:24", which may indicate that generation time of the fused result data/the new first application card 721 is 02:24 on August 14.

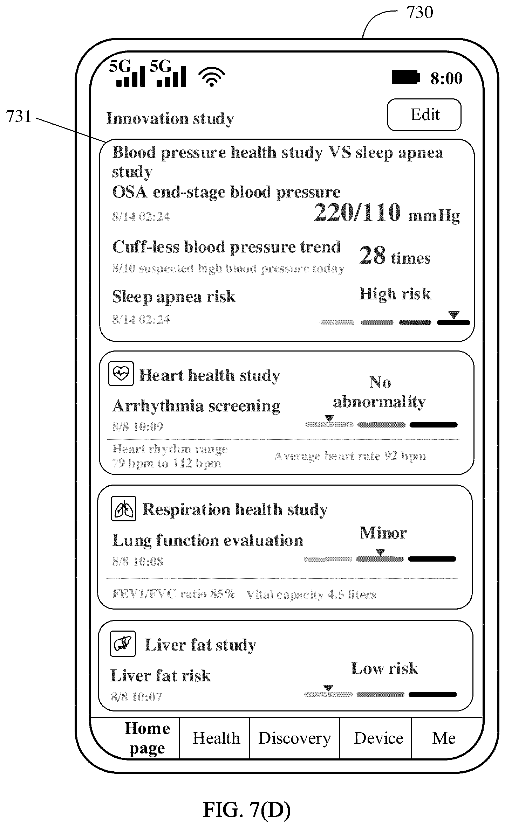

This is not limited to the foregoing implementation. In another implementation, the electronic device 100 may further display result data of an original application card in the fused application card. In some examples, the electronic device 100 may display, in response to the user operation for the new first application card 711 and the new first application card 712 in the user interface 710 shown in FIG. 7(B), a user interface 730 shown in FIG. 7(D). As shown in FIG. 7(D), the user interface 730 is similar to the user interface 720, and a difference lies in that an application card obtained through fusion of the new first application card 711 and the new first application card 712 is different, that is, a new first application card 731 in the user interface 730 is different from the new first application card 721 in the user interface 720. The new first application card 731 may include result data of three types of physiological parameters. For example, the result data of the blood pressure, the result data of the sleep apnea, and the result data obtained through fusion of the blood pressure and the sleep apnea. The result data of the blood pressure is result data indicated by the new first application card 711 in the user interface 710, the result data of the sleep apnea is result data indicated by the new first application card 712 in the user interface 710, and the result data obtained through fusion of the blood pressure and the sleep apnea is result data indicated by the new first application card 721 in the user interface 720. Details are not described again.

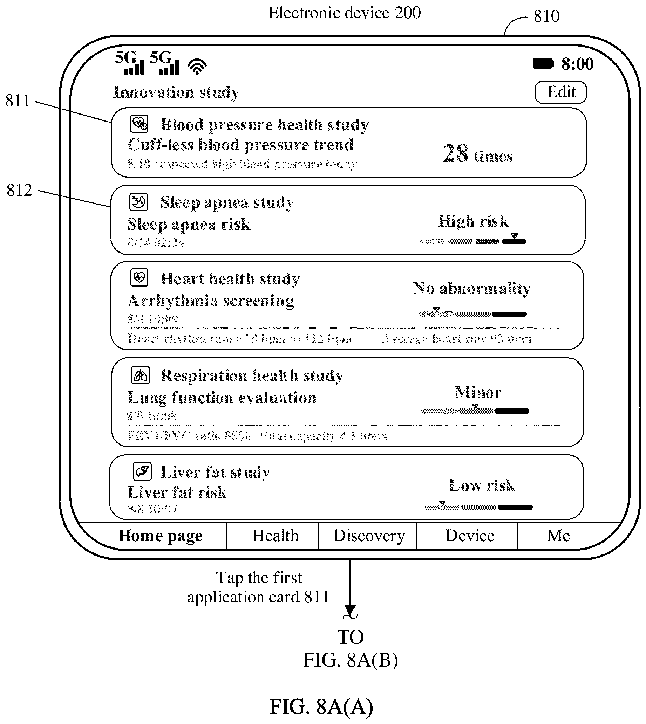

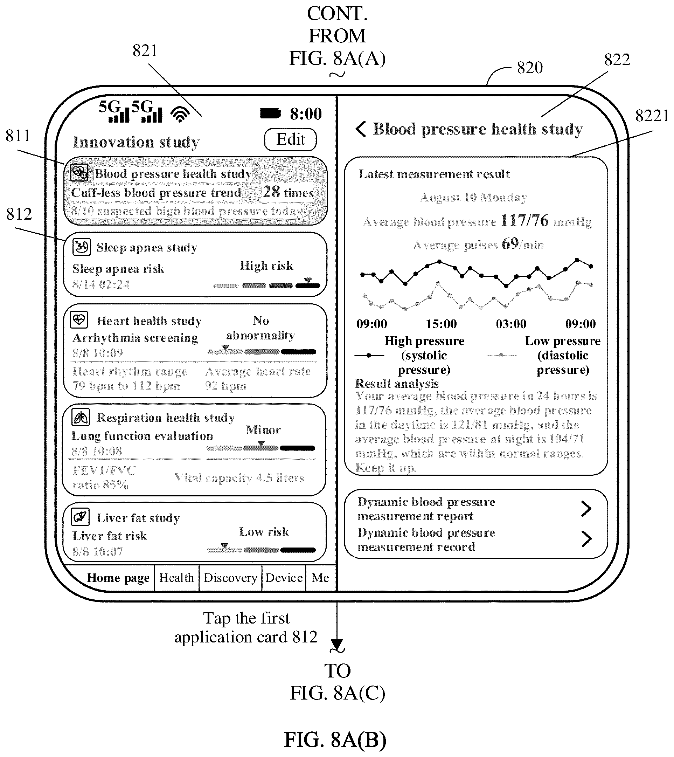

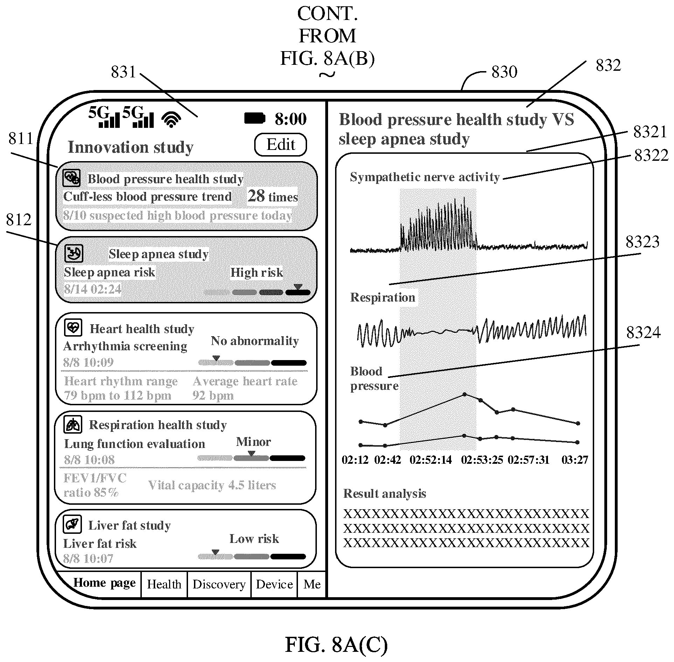

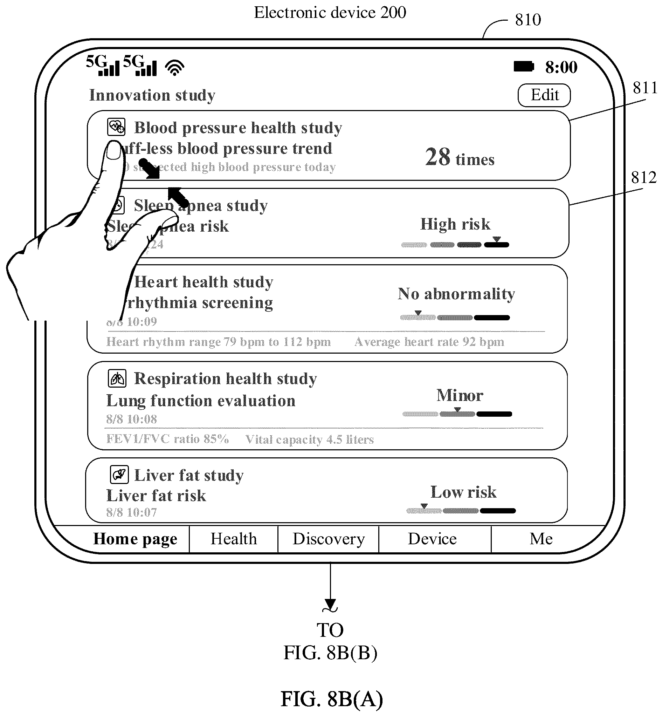

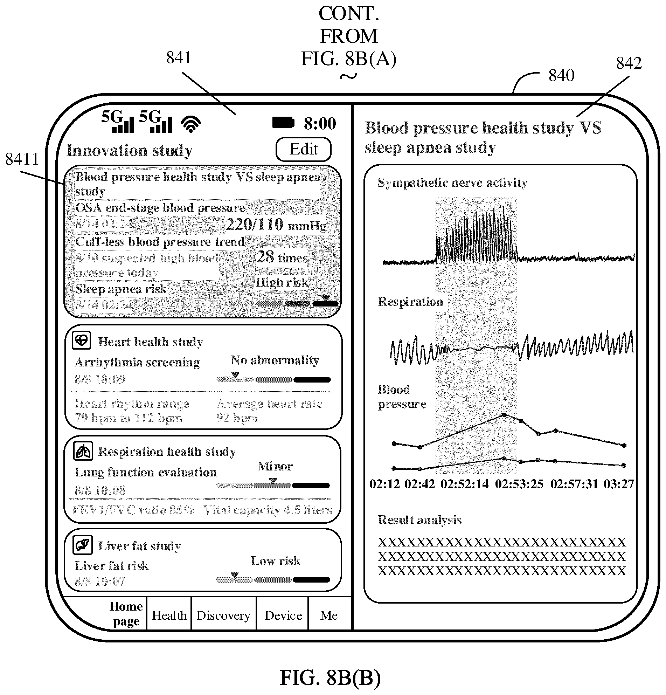

The electronic device is not limited to the foregoing examples. In some other examples, the electronic device may alternatively be a foldable electronic device, for example, the electronic device 200 shown in FIG. 3. For specific examples, refer to FIG. 8A and FIG. 8B.

As shown in FIG. 8A(A), the electronic device 200 may display a user interface 810. The user interface 810 may include a plurality of new first application cards, for example, a new first application card 811, a new first application card 812, and another new first application card. The user interface 810 is similar to the user interface 710 shown in FIG. 7(A). Details are not described again. In an implementation, in response to a user operation (for example, the user operation is a touch operation) for the new first application card 811 in the user interface 810, the electronic device 200 may select the new first application card 811, and display a detailed interface of the application card. For a specific example, refer to a user interface 820 shown in FIG. 8A(B).

As shown in FIG. 8A(B), the user interface 820 may include two display areas, for example, a display area 821 and a display area 822. The display area 821 is similar to the user interface 810 shown in FIG. 8A(A), and a difference lies in that the new first application card 811 in the display area 821 is in a selected state, and the display area 822 is used to display the detailed interface of the selected new first application card 811. The display area 822 may include a plurality of application cards, for example, an application card 8221. The application card 8221 may be used to display result data of blood pressure. In an implementation, the electronic device 200 may select the new first application card 812 and the new first application card 811 in response to a user operation (for example, the user operation is a touch operation) for the new first application card 812 in the user interface 820, and perform data fusion on abnormal data in measurement data of the blood pressure indicated by the new first application card 812 and measurement data of sleep apnea indicated by the new first application card 811, to obtain fused result data. In this case, the electronic device 200 may display a user interface 830 shown in FIG. 8A(C).

As shown in FIG. 8A(C), the user interface 830 may include two display areas, for example, a display area 831 and a display area 832. The display area 831 is similar to the display area 821 in the user interface 820 shown in FIG. 8A(B), and a difference lies in that the new first application card 812 in the display area 831 is in the selected state. The display area 832 is used to display a detailed interface of the fused result data. The display area 832 may include an application card 8321. The application card 8321 may include text content 8322, text content 8323, and text content 8324. The text content 8322 includes characters "Sympathetic nerve activity". A curve chart is displayed below the text content 8322, and may indicate the result data obtained through fusion of the blood pressure and the sleep apnea. Data displayed in a gray area is data obtained through data fusion of abnormal data in the two types of data: the blood pressure and the sleep apnea. The text content 8323 includes a character "Respiration". A curve chart is displayed below the text content 8323, and may indicate result data of the sleep apnea. Data displayed in a gray area is abnormal data in the measurement data of the sleep apnea. The text content 8324 includes characters "Blood pressure". A curve chart is displayed below the text content 8324, and may indicate the result data of the blood pressure. Data displayed in a gray area is abnormal data in the measurement data of the blood pressure.

This is not limited to the foregoing implementation. In another implementation, as shown in FIG. 8B(A), the electronic device 200 may receive a user operation (the user operation is, for example, a touch operation, and the touch operation is, for example, a pinch operation) for the new first application card 811 and the new first application card 812 in the user interface 810. In response to the user operation, the electronic device 200 may perform data fusion on the abnormal data in the two types of data: the measurement data of the blood pressure and the measurement data of the sleep apnea, to obtain fused result data, fuse the new first application card 811 and the new first application card 812, and display the fused result data in a fused application card, and optionally, the result data of the original application cards. In this case, the electronic device 200 may display a user interface 840 shown in FIG. 8B(B).

As shown in FIG. 8B(B), the user interface 840 may include two display areas, for example, a display area 841 and a display area 842. The display area 841 is similar to the user interface 730 shown in FIG. 7(D), and a difference lies in that a new first application card 8411 in the display area 841 is in the selected state. The display area 841 is similar to the display area 832 in the user interface 830 shown in FIG. 8A(C). Details are not described again. This is not limited to the foregoing implementation. In another implementation, the electronic device 200 may alternatively display only the fused result data in the fused application card. For example, the new first application card 8411 in the display area 841 may be replaced with the new first application card 721 in the user interface 720 shown in FIG. 7(C) for display. In addition, the new first application card 721 is in the selected state.

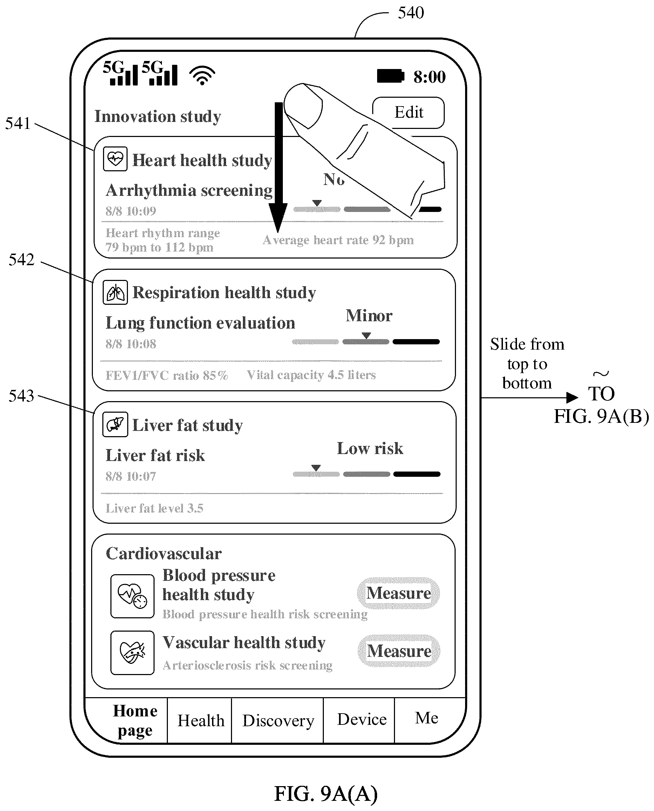

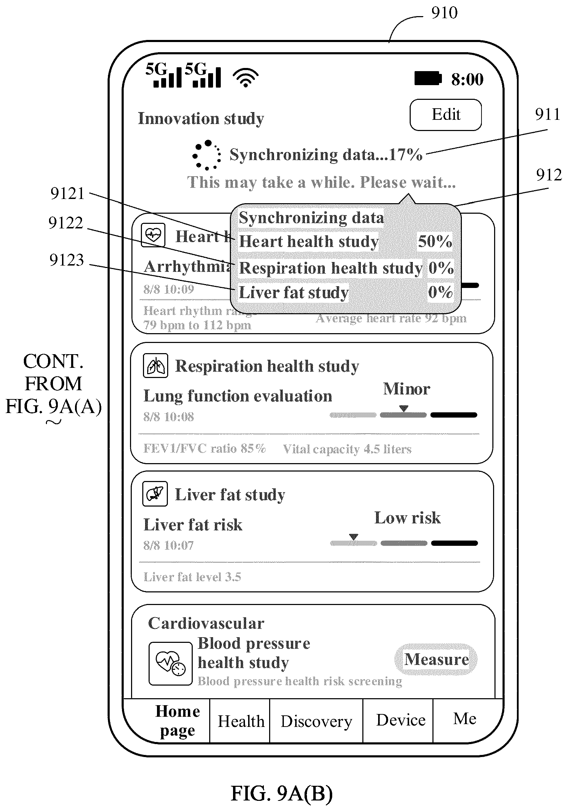

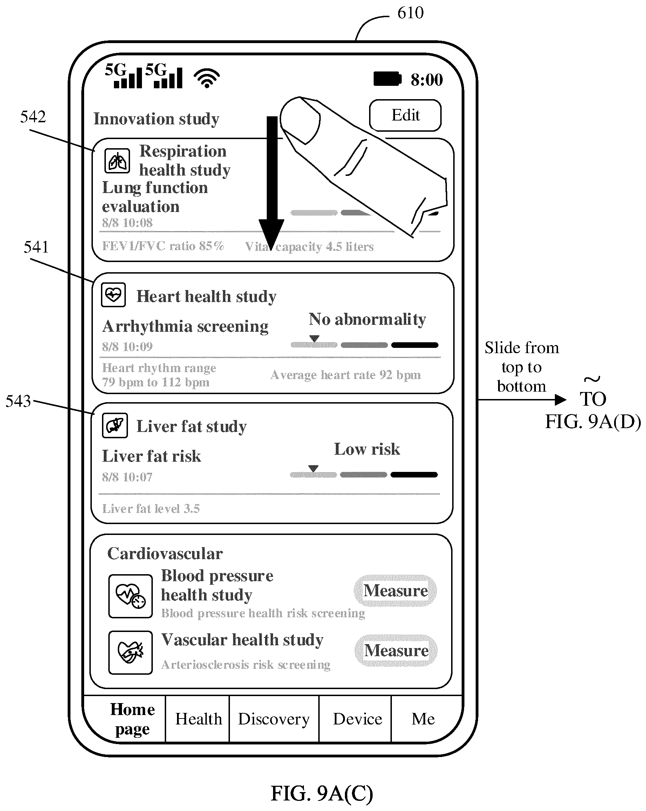

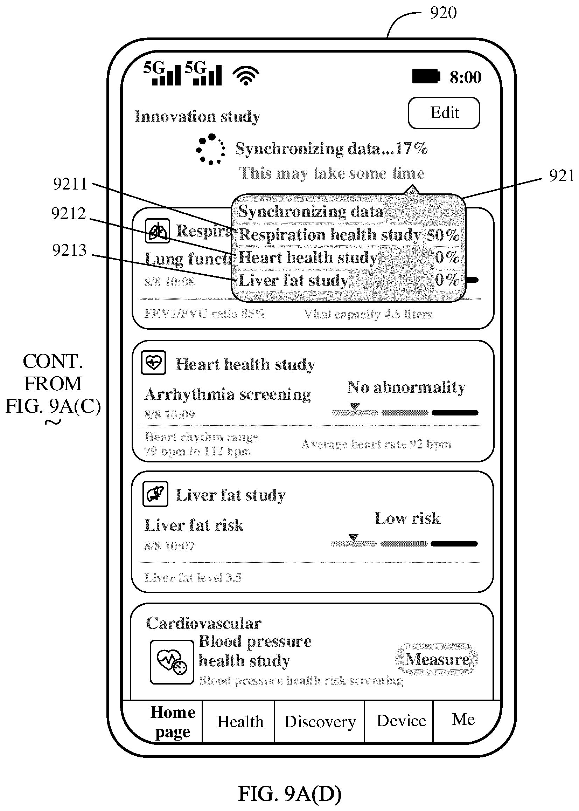

In an implementation, after the implementation shown in FIG. 5(D), the electronic device 100 may receive a user operation, and obtain, in response to the user operation, measurement data of a physiological parameter corresponding to a new first application card, to update result data corresponding to the new first application card. In some examples, the electronic device 100 may receive a user operation (for example, the user operation is a sliding operation from top to bottom) for a user interface 540 shown in FIG. 9A(A). In response to the user operation, the electronic device 100 may sequentially obtain measurement data of a heart rate indicated by a new first application card 541, measurement data of a lung function indicated by a new first application card 542, and measurement data of liver fat indicated by a new first application card 543. In this case, the electronic device 100 may display a user interface 910 shown in FIG. 9A(B).

As shown in FIG. 9A(B), the user interface 910 may include text content 911 and a control 912. The control 912 includes text content 9121, text content 9122, and text content 9123. The text content 9121 includes characters "Heart health study", and a character "50%" is displayed on the right, which may represent that progress of currently obtaining the measurement data of the heart rate is 50%. The text content 9122 includes characters "Respiration health study", and a character "0%" is displayed on the right, which may represent that progress of currently obtaining the measurement data of the lung function is 0%. The text content 9123 includes characters "Liver fat study", and a character "0%" is displayed on the right, which may represent that current progress of obtaining the measurement data of the liver fat is 0%. The text content 911 includes characters "Synchronizing data...17%", which may represent that total progress of currently obtaining measurement data of a plurality of types of physiological parameters (namely, the heart rate, the lung function, and the liver fat) is 17%.

Then, after the electronic device 100 obtains measurement data of physiological parameters corresponding to new first application cards, for example, sequentially obtains the measurement data of the heart rate, the measurement data of the lung function, and the measurement data of the liver fat, the electronic device 100 may separately analyze the three types of measurement data, generate three types of result data, and display the three types of generated result data in the corresponding new first application cards, to update result data corresponding to the new first application cards.

In an implementation, an order in which the electronic device 100 obtains the measurement data of the plurality of types of physiological parameters corresponding to the plurality of new first application cards is consistent with a top-bottom display order of the plurality of new first application cards. In some examples, after the implementation shown in FIG. 6(B), the electronic device 100 may receive a user operation (for example, the user operation is a sliding operation from top to bottom) for a user interface 610 shown in FIG. 9A(C). In response to the user operation, the electronic device 100 may sequentially obtain the measurement data of the lung function indicated by the new first application card 542, the measurement data of the heart rate indicated by the new first application card 541, and the measurement data of the liver fat indicated by the new first application card 543. In this case, the electronic device 100 may display a user interface 920 shown in FIG. 9A(D).

As shown in FIG. 9A(D), the user interface 920 is similar to the user interface 910 shown in FIG. A(B), and a difference lies in that a top-bottom display order of the three pieces of text content included in the control 921 in the user interface 920 is different from a top-bottom display order of the three pieces of text content included in the control 912 in the user interface 910. The control 921 may include text content 9211, text content 9212, and text content 9213. The text content 9211 includes characters "Respiration health study", and a character "50%" is displayed on the right, which may represent that progress of currently obtaining the measurement data of the lung function is 50%. The text content 9212 includes characters "Heart health study", and a character "0%" is displayed on the right, which may represent that progress of currently obtaining the measurement data of the heart rate is 0%. The text content 9123 includes characters "Liver fat study", and a character "0%" is displayed on the right, which may represent that current progress of obtaining the measurement data of the liver fat is 0%.

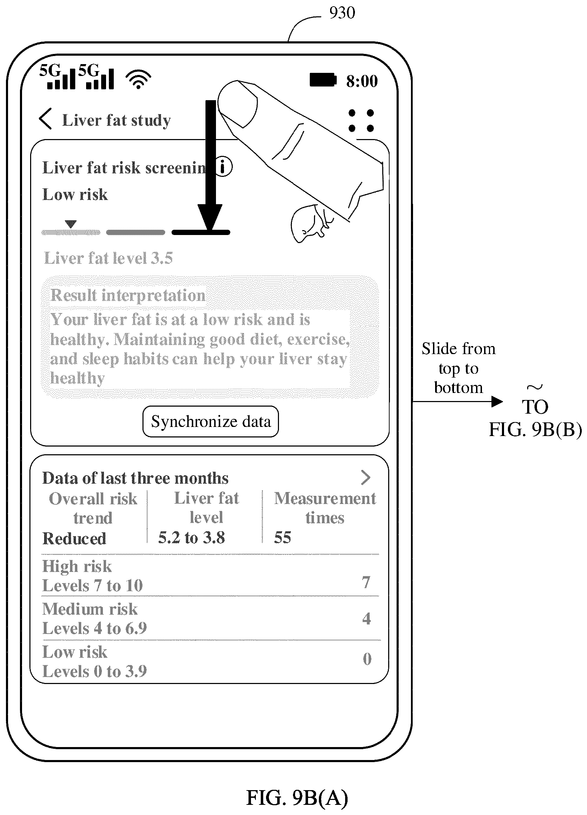

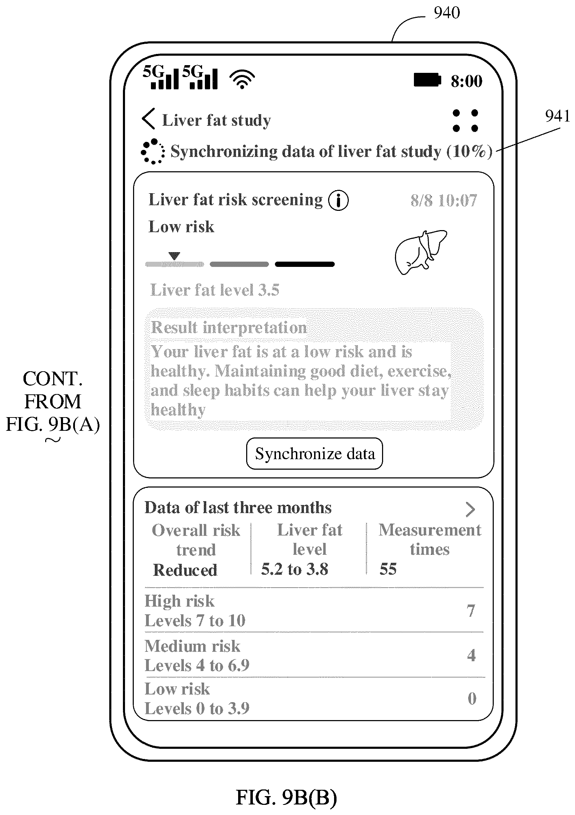

This is not limited to the foregoing example. In some other examples, when the electronic device 100 displays a detailed interface of any new first application card, the electronic device 100 may receive a user operation for the detailed interface. In response to the user operation, the electronic device 100 may obtain measurement data of a physiological parameter corresponding to the new first application card, to update result data corresponding to the new first application card. For example, the electronic device 100 may display a user interface 930 shown in FIG. 9B(A). The user interface 930 may be a detailed interface of the liver fat. The electronic device 100 may receive a user operation (for example, the user operation is a sliding operation from top to bottom) for the user interface 930. In response to the user operation, the electronic device 100 may obtain the measurement data of the liver fat. In this case, the electronic device 100 may display a user interface 940 shown in FIG. 9B(B). The user interface 940 may include text content 941. The text content 941 includes characters "Synchronizing data of liver fat study (10%)", which may represent that the measurement data of the liver fat is being obtained currently, and obtaining progress is 10%.

This is not limited to a case in which the electronic device 100 obtains, in the detailed interface of the new first application card, the measurement data of the physiological parameter corresponding to the application card. In some other examples, when the electronic device 100 displays a detailed interface of any new first application card, and the electronic device 100 is obtaining measurement data of a physiological parameter, the electronic device 100 may display, in the detailed interface in response to a user operation for the detailed interface, progress information of obtaining the physiological parameter. For example, when the electronic device 100 displays the user interface 930 shown in FIG. 9B(A), the electronic device 100 is obtaining the measurement data of the heart rate. In this case, the electronic device 100 may display a user interface similar to the user interface 940 in response to a user operation (for example, the user operation is a sliding operation from top to bottom) for the user interface 930. A difference lies in that the user interface does not include text content 941, but includes progress information. The progress information includes characters "Synchronizing data of heart health study (50%). Synchronize data of liver fat study later", which may indicate that the electronic device 100 is currently obtaining the measurement data of the heart rate, and obtaining progress is 50%.

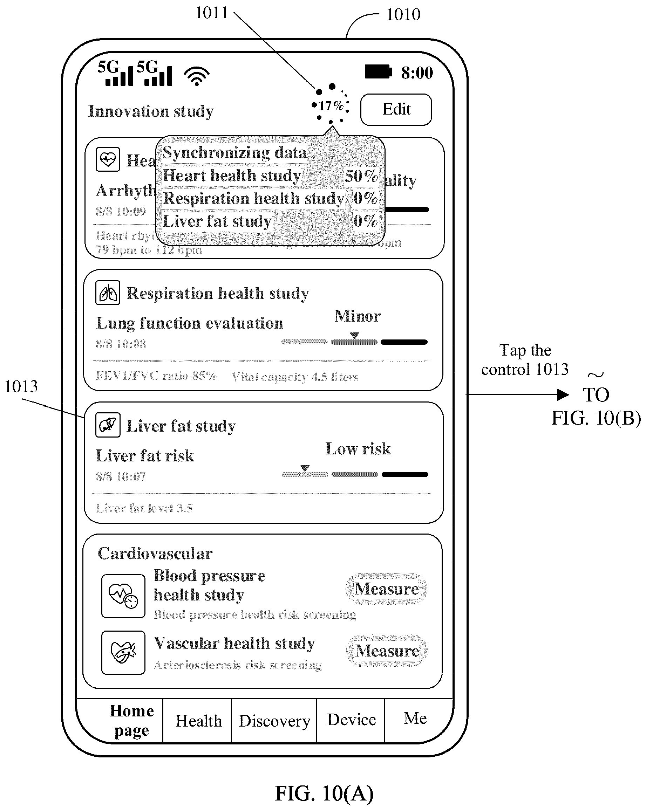

This is not limited to the foregoing example. In some other examples, the electronic device 100 may obtain measurement data of a physiological parameter when a health application is started, to update result data of the physiological parameter. For example, the electronic device 100 may receive a user operation (for example, the user operation is a touch operation) for a control of the health application. In response to the user operation, the electronic device 100 may start the health application, and obtain measurement data of physiological parameters corresponding to new first application cards in a preset order (it is assumed that the preset order is the top-bottom display order of the three new first application cards in the user interface 910 shown in FIG. 9A(B)). In this case, the electronic device 100 may display a user interface 1010 shown in FIG. 10(A). The user interface 1010 is similar to the user interface 910 shown in FIG. 9A(B), and a difference lies in that the user interface 1010 includes a control 1011 but does not include the text content 911. A character "17%" is displayed on the control 1011, which may represent that total progress of currently obtaining measurement data of a plurality of types of physiological parameters (namely, the heart rate, the lung function, and the liver fat) is 17%.

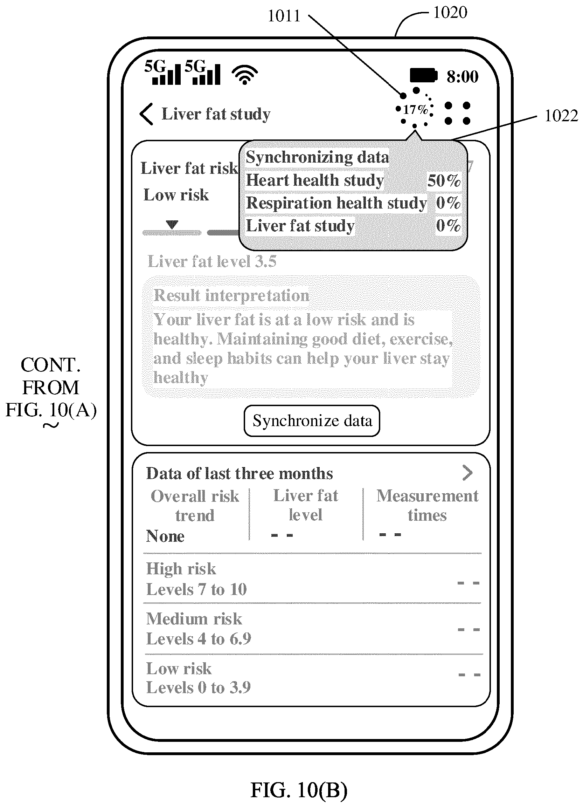

In an implementation, the electronic device 100 may display a detailed interface of a new first application card 1013 in response to a user operation (for example, the user operation is a touch operation) for the new first application card 1013 in the user interface 1010. For a specific example, refer to the user interface 1020 shown in FIG. 10(B). The user interface 1020 may include a control 1011 and a control 1022. The control 1022 is similar to the control 912 in the user interface 910 shown in FIG. 9A(B). Details are not described again.

This is not limited to the foregoing example. In some other examples, the electronic device 100 may alternatively automatically update the result data of the physiological parameter, for example, update the result data of the physiological parameter at an interval of preset duration, and may display FIG. 10(A) or FIG. 10(B) during the update.

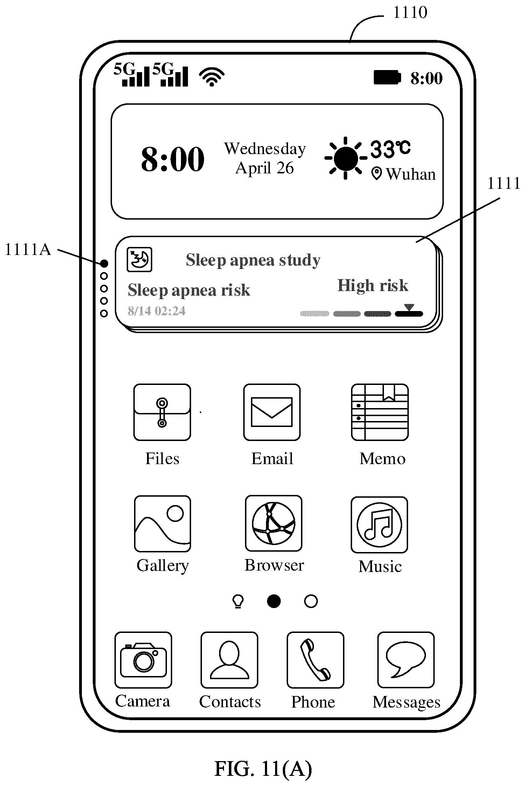

This is not limited to the foregoing implementation in which the result data of the physiological parameter is displayed in the application card. In another implementation, the electronic device 100 may alternatively display the result data of the physiological parameter in a card, for example, a card 1111 on a home screen shown in FIG. 11(A) to FIG. 11(C).

As shown in FIG. 11(A), the electronic device 100 may display a user interface 1110. The user interface 1110 is the home screen of the electronic device 100. The user interface 1110 may include the card 1111. The card 1111 may include result data of sleep apnea. The card 1111 is similar to the new first application card 712 in the user interface 710 shown in FIG. 7(A). Details are not described again. An indicator 1111A is displayed on the left of the card 1111, and the indicator 1111A may indicate that result data corresponding to a first (topmost) card is currently displayed in the home screen.

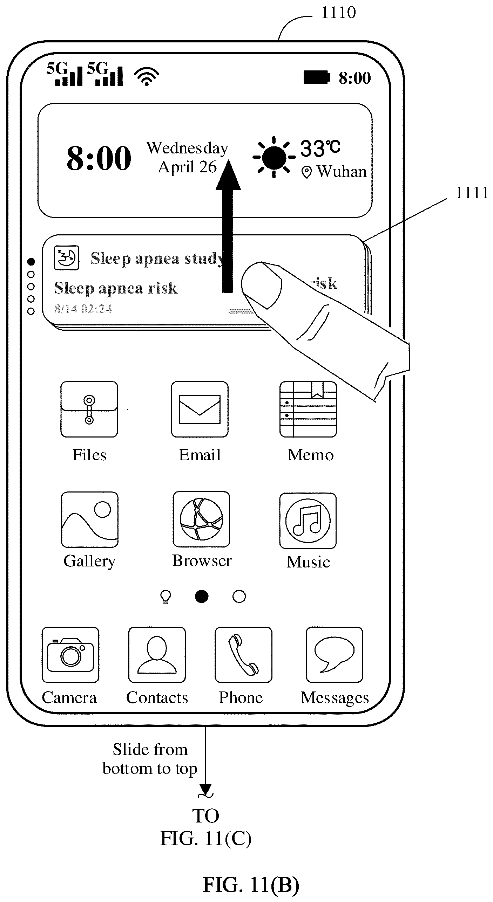

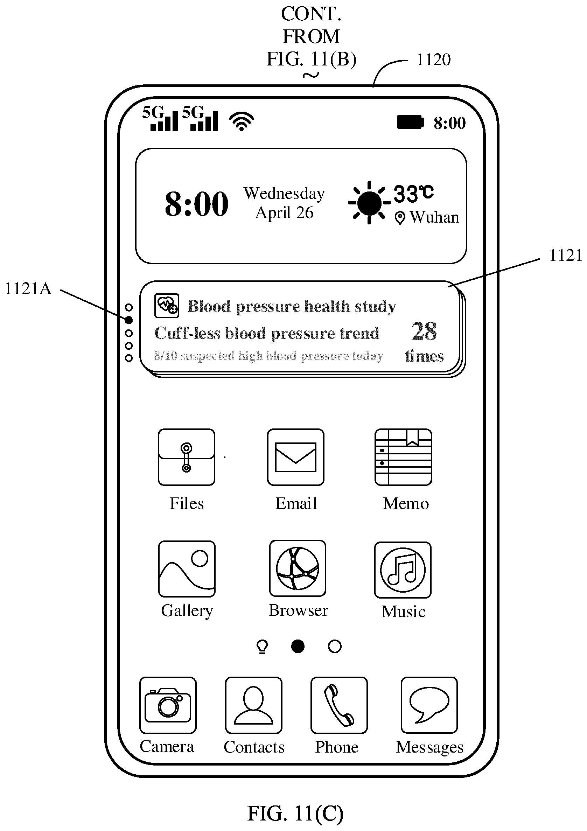

In an implementation, the electronic device 100 may switch a displayed card in response to a user operation (for example, the user operation is a sliding operation from bottom to top) for the card 1111 in the user interface 1110 shown in FIG. 11(B). In this case, the electronic device 100 may display a user interface 1120 shown in FIG. 11(C). The user interface 1120 is similar to the user interface 1110, and a difference lies in that a card 1121 in the user interface 1120 is different from the card 1111 in the user interface 1110. The card 1121 may include result data of blood pressure, and an indicator 1121A is displayed on the left of the card 1121. The indicator 1121A may indicate that result data corresponding to a second card (from top to bottom) is currently displayed in the home screen.

This is not limited to the foregoing implementation. In another implementation, the electronic device 100 may receive a user operation for the new first application card, and pin/mark the new first application card in response to the user operation. In some examples, the electronic device 100 may receive a user operation (the user operation is, for example, a touch operation, and the touch operation is, for example, a touch and hold operation) for the new first application card 543 in the user interface 540 shown in FIG. 5(D), and display a menu corresponding to the new first application card 543 in response to the user operation. The menu may include a plurality of options, for example, a pin option and a mark option. For example, the electronic device 100 may move, in response to a user operation (for example, the user operation is a touch operation) for the pin option, a display location of the new first application card 543 to the top of all the new first application cards in the user interface 540. In this case, the application cards in the user interface displayed by the electronic device 100 are displayed from top to bottom in the following order: the new first application card 543, the new first application card 541, the new first application card 542, and the first application card 544. For another example, the electronic device 100 may mark the new first application card 543 in response to a user operation (for example, the user operation is a touch operation) for the mark option. In this case, the electronic device 100 may display a user interface similar to the user interface 540, and a difference lies in that an upper right corner of the new first application card 543 in the user interface further includes a mark symbol. The mark symbol may represent that the new first application card 543 is marked.

This is not limited to the foregoing implementation. In another implementation, the electronic device 100 may receive a user operation for at least one new first application card, and in response to the user operation, add the at least one new first application card to the home screen/a leftmost screen for display, or delete the at least one new first application card and related data. In some examples, the user interface 540 shown in FIG. 5(D) may further include a management control. The electronic device 100 may receive a user operation (for example, the user operation is a touch operation) for the management control, and in response to the user operation, the electronic device 100 may display a user interface similar to the user interface 540. A difference lies in that a selection control is further displayed in any new first application card in the user interface, and the selection control may be used to select a corresponding new first application card or cancel the selection. A taskbar is further displayed at the bottom of the user interface, and the taskbar includes a plurality of controls, for example, an add control and a delete control. The electronic device 100 may select the new first application card 541 and the new first application card 542 in response to user operations (for example, the user operation is a touch operation) for a selection control corresponding to the new first application card 541 and a selection control corresponding to the new first application card 542. Then, in response to user operations (for example, the user operation is a touch operation) for add controls, the electronic device 100 may add the new first application card 541 and the new first application card 542 that are selected to the home screen/leftmost screen for display. In this case, the home screen/leftmost screen of the electronic device 100 may display two cards, and the two cards may respectively indicate result data included in the two new first application cards.

In some other examples, it is assumed that the new first application card 541 is selected. Then, the electronic device 100 may delete the new first application card 541 and related data in response to a user operation (for example, the user operation is a touch operation) for the delete control, and optionally, disable the measurement function of the heart rate corresponding to the new first application card 541. In this case, the electronic device 100 may display a user interface after the deletion. The user interface may include the new first application card 542 and the new first application card 543, and does not include the new first application card 541. In addition, the first application card 544 in the user interface further includes the second application card 5111 in the user interface 510 shown in FIG. 5(A). It may be understood that after the electronic device 100 deletes the new first application card and the related data (for example, including but not limited to the measurement data and the result data of the physiological parameter corresponding to the new first application card), and disables the measurement function of the physiological parameter, the electronic device 100 switches the new first application card corresponding to the physiological parameter to a second application card for display, and displays the second application card in the first application card to which the physiological parameter belongs.

This is not limited to the foregoing example. In some other examples, the electronic device 100 may receive a user operation for the home screen/leftmost screen, and add at least one new first application card to the home screen/leftmost screen in response to the user operation. For example, the electronic device 100 may receive a user operation (the user operation is, for example, a touch operation, and the touch operation is, for example, a touch and hold operation) for the home screen, and display, in response to the user operation, a selection interface for adding a widget/card. The selection interface includes controls of a plurality of applications, for example, a control of the health application. The electronic device 100 may add, in response to a user operation (for example, the user operation is a touch operation) for the control of the health application, any one of at least one new first application card in the health application to the home screen for display.

The following describes a display method provided in an embodiment of this application.

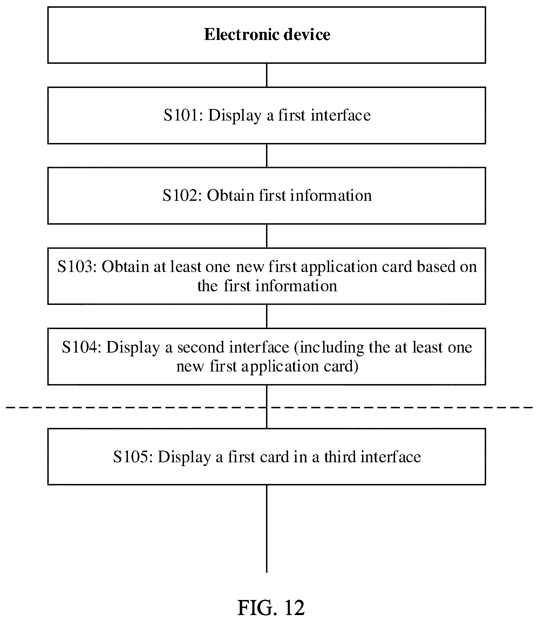

FIG. 12 is a schematic flowchart of a display method according to an embodiment of this application. The method may be applied to the electronic device 100 shown in FIG. 1. The method may be applied to the electronic device 100 shown in FIG. 2. The method may be applied to the electronic device 200 shown in FIG. 3. The method may include but is not limited to the following steps.

S101: An electronic device displays a first interface.

In an implementation, the first interface may be a user interface of a health application.

In an implementation, the first interface may include at least one first application card. One first application card may indicate one type of physiological category. Each first application card may include at least one second application card. A measurement result of a physiological parameter is not displayed in the second application card. The second application card may be used to trigger measurement of a corresponding physiological parameter. It may be understood that the electronic device provides, for a user by using the second application card, an entry of a measurement function of the corresponding physiological parameter, and the second application card is a card that is displayed when the measurement function corresponding to the application card is not enabled/that does not include a measurement result of the corresponding physiological parameter. One type of physiological parameter indicated by any second application card may belong to one type of physiological category, and second application cards belonging to a same physiological category may be displayed in one first application card. In some examples, the physiological parameter includes but is not limited to, for example, a heart rate, blood pressure, vascular sclerosis, a lung function, sleep apnea, altitude sickness, blood glucose, and liver fat. A physiological category to which the heart rate, the blood pressure, and the vascular sclerosis belong is cardiovascular, a physiological category to which the lung function, the sleep apnea, and the altitude sickness belong is respiration, a physiological category to which the blood glucose belongs is endocrine, and a physiological category to which the liver fat belongs is fitness. For example, the first interface is the user interface 510 shown in FIG. 5(A).

S102: The electronic device obtains first information.

In an implementation, the first information may include measurement data of at least one type of physiological parameter of the user, for example, including but not limited to the heart rate, the blood pressure, the vascular cirrhosis, the lung function, the sleep apnea, the altitude sickness, the blood glucose, and the liver fat.

In an implementation, the electronic device may receive, in response to a user operation, measurement data that is of the at least one type of physiological parameter of the user and that is measured and sent by a connected wearable device. In another implementation, the electronic device may obtain, in response to a user operation, measurement data of the at least one type of physiological parameter of the user from an application (for example, a sports health app) related to the at least one type of physiological parameter. In another implementation, the electronic device may send request information to a cloud in response to a user operation, to obtain measurement data that is of the at least one type of physiological parameter of the user and that is stored in the cloud. For a specific implementation process, refer to the implementation shown in FIG. 5(B). Details are not described again.

S103: The electronic device obtains at least one new first application card based on the first information.

In an implementation, the electronic device may generate result data of the at least one type of physiological parameter based on the measurement data of the at least one type of physiological parameter, to obtain the at least one new first application card. The new first application card may be used to display the result data of the corresponding physiological parameter. In some examples, the first information includes, for example, measurement data of the liver fat. The electronic device may analyze the measurement data of the liver fat, to generate result data of the liver fat, so as to obtain a new first application card, for example, the new first application card 531 in the user interface 530 shown in FIG. 5(C).

In an implementation, each new first application card may be used to display information about a physiological parameter and result data of the physiological parameter. In some examples, the result data of the physiological parameter may include but is not limited to information such as a measurement result of the physiological parameter, generation time of the result data, a risk level of the measurement result, and measurement time of the measurement data. For example, the new first application card 531 in the user interface 530 shown in FIG. 5(C) may include information about the heart rate (namely, information indicated by the title "Heart health study" and the characters "Arrhythmia screening"), a measurement result of the heart rate (namely, a result represented by the characters "Heart rate range 79 bpm to 112 bpm" and "Average heart rate 92 bpm" included in the text content 5312), generation time (namely, the characters "8/8 10:09" included in the text content 5311) of result data, and a risk level (namely, the risk level indicated by the control 5313) of the measurement result.

S104: The electronic device displays a second interface (including the at least one new first application card).

In an implementation, the electronic device may display the second interface. The second interface may be a user interface of the health application. The second interface may include the at least one new first application card. The at least one new first application card is displayed above the at least one first application card. In some examples, the electronic device may display the at least one new first application card on the top of the second interface, and the electronic device may delete the second application card related to the measurement data of the at least one type of physiological parameter included in the first information. The second application card may indicate the at least one type of physiological parameter in the first information. It may be understood that the electronic device may cancel displaying the second application card that is in the first application card and that is related to the first information. For example, the first interface is the user interface 510 shown in FIG. 5(A). After obtaining the measurement data of the heart rate, the measurement data of the lung function, and the measurement data of the liver fat, the electronic device generates three new first application cards. The three new first application cards may be displayed on the top of the second interface. For example, the second interface is the user interface 540 shown in FIG. 5(D). In this case, the second application card 5111 indicating the measurement function of the heart rate is no longer displayed in the first application card 544 in the user interface 540, and the second application card 5121 indicating the measurement function of the lung function and the second application card 5131 indicating the measurement function of the liver fat are no longer displayed in the user interface 540. Optionally, because the first application card 513 includes only one second application card 5131, the first application card 513 and the second application card 5131 included in the first application card 513 are no longer displayed in the second interface.

In an implementation, the electronic device may determine a first display order according to a first preset rule, and display the at least one new first application card in the second interface in the first display order. In some examples, the first preset rule may include but is not limited to time (for example, generation time of result data, generation time of the new first application card, and measurement time of measurement data), risk levels of measurement results, a display order of first application cards corresponding to physiological categories, and the like. For example, generation time of the result data of the heart rate is 10:09 on August 8, generation time of the result data of the lung function is 10:08 on August 8, and generation time of the result data of the liver fat is 10:07 on August 8. The electronic device may determine, based on the generation time of the result data of the three types of physiological parameters (it is assumed that the result data of the three types of physiological parameters is sequentially sorted from bottom to top by time from early to late), that the first display order (a top-bottom display order) of the corresponding three new first application cards is as follows: a new first application card corresponding to the heart rate, a new first application card corresponding to the lung function, and a new first application card corresponding to the liver fat. An interface for displaying the three new first application cards in the first display order is the user interface 540 shown in FIG. 5(D).

Optionally, when displaying the second interface, the electronic device may further prompt, in a manner like displaying prompt information in a notification bar, light prompting, voice prompting, or displaying prompt information in a pop-up window, the user that the at least one new first application card is currently displayed, that is, the result data of the physiological parameter can be currently viewed.

In an implementation, the method further includes the following step.

S105: The electronic device displays a first card in a third interface.

In an implementation, S 105 is an optional step.

In an implementation, the third interface may be an interface of the electronic device other than the user interface of the health application, for example, any page of a home screen, a lock screen interface, a leftmost screen, or a notification interface.

In an implementation, the electronic device may display the first card on the third interface. The first card may be any one of the at least one new first application card, and the first card may include result data of a corresponding physiological parameter.

In an implementation, the electronic device may determine whether the first card is displayed in the third interface. When the first card is not displayed in the third interface, the electronic device may autonomously display the first card on the third interface. For example, in response to a touch operation for a return control in the second interface, the electronic device may exit the health application when determining that the first card is not displayed in the third interface. In this case, the electronic device may display the second interface. The second interface is, for example, the user interface 1110 shown in FIG. 11(A). The first card is, for example, the card 1111 in the user interface 1110, and the first card may include result data of the sleep apnea.

In an implementation, the first card may include a plurality of cards that can be switched, and one card that can be switched may be used to display result data of one type of physiological parameter. The electronic device may switch, in response to a user operation, a card displayed in the third interface, to switch result data of a physiological parameter for display. In some examples, the card before switching is, for example, the card 1111 in the user interface 1110 shown in FIG. 11(B). The card 1111 may include the result data of the sleep apnea. In response to a sliding operation from bottom to top for the card 1111, the electronic device may switch, the card 1111 to the card 1121 (namely, a card obtained after switching) in the user interface 1120 shown in FIG. 11(C), and the card 1121 may include result data of the blood pressure.

In an implementation, when the first card includes the plurality of cards that can be switched, the plurality of cards that can be switched may be stacked and sorted according to a second preset rule. In some examples, the second preset rule may include but is not limited to risk levels of measurement results, generation time of result data, measurement time of measurement data, and the like. For example, a risk level of a measurement result of the heart rate is a high risk, a risk level of a measurement result of the lung function is a medium risk, and a risk level of a measurement result of the liver fat is a low risk, the electronic device may obtain, based on the risk levels of the measurement results of the three types of physiological parameters (it is assumed that stacking and sorting is performed from top to bottom based on the risk levels from high to low/from severe to not severe), a top-bottom order of three cards: result data of the heart rate, result data of the lung function, and result data of the liver fat.

In an implementation, a quantity of the plurality of cards that can be switched and that are included in the first card may be less than or equal to a preset threshold. When a quantity of a plurality of new first application cards in the health application is greater than the preset threshold, the electronic device may select, according to a third preset rule, physiological parameters whose quantity is the preset threshold from a plurality of types of physiological parameters corresponding to the plurality of new first application cards, and display the selected physiological parameters by using the plurality of cards that can be switched. One card that can be switched corresponds to result data of one type of physiological parameter. In some examples, the third preset rule may include but is not limited to being marked/pinned by the user, a risk level of a measurement result, generation time of result data, measurement time of measurement data, generation time of the new first application card, and the like.

This is not limited to the implementation in which the electronic device displays the first card in the third interface. In another implementation, the electronic device may further send the result data of the at least one type of physiological parameter to the wearable device. After receiving the result data of the at least one type of physiological parameter, the wearable device may display the result data of the at least one type of physiological parameter in a form of card. The card displayed by the wearable device on a display is, for example, the card 1111 in the user interface 1110 shown in FIG. 11(A) and FIG. 11(B). Optionally, the wearable device may switch a displayed card in response to a user operation for the card. A card obtained after switching is, for example, the card 1121 in the user interface 1120 shown in FIG. 11(C).

In the method shown in FIG. 12, after obtaining the measurement data of the at least one type of physiological parameter of the user, the electronic device can generate the at least one new first application card based on the measurement data, and display the at least one new first application card on the top of the user interface of the application. The user may directly obtain the measurement result of the at least one type of physiological parameter by using the at least one new first application card displayed on the top of the user interface of the application, and does not need to perform operations such as starting a plurality of apps, switching pages a plurality of times, and dragging and sliding a scroll bar on a page a plurality of times. Therefore, according to the foregoing method, the user can quickly and conveniently obtain the measurement result of the physiological parameter, operations that need to be performed by the user to obtain the measurement result are reduced, and user operations are greatly reduced especially when there are a large quantity of physiological parameters. This effectively improves efficiency of obtaining information by the user, and improves user experience.

In addition, the electronic device can display, in a form of card, the result data of the at least one type of physiological parameter in the interface other than the user interface of the application, for example, the home screen, the leftmost screen, the lock screen interface, or the notification interface. In this way, when the user interface of the application is not displayed, the user can still view information such as the measurement result of the physiological parameter in time, thereby effectively improving efficiency of obtaining information by the user and improving user experience.

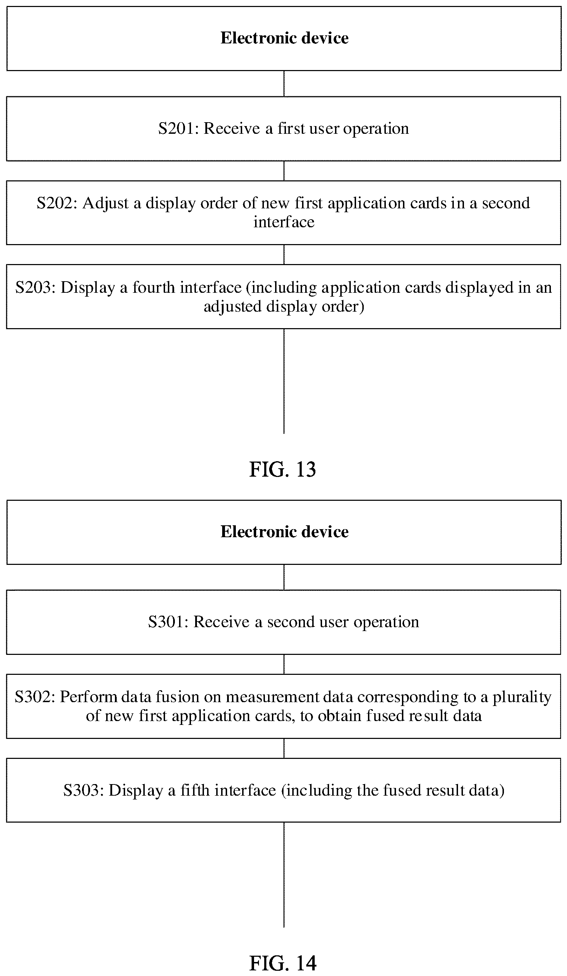

FIG. 13 is a schematic flowchart of another display method according to an embodiment of this application. The method may be applied to the electronic device 100 shown in FIG. 1. The method may be applied to the electronic device 100 shown in FIG. 2. The method may be applied to the electronic device 200 shown in FIG. 3. The method may include but is not limited to the following steps.

S201: An electronic device receives a first user operation.

In an implementation, before S201 in FIG. 13, S101 to S104 in FIG. 12 are further included.

S202: The electronic device adjusts a display order of new first application cards in a second interface.

In an implementation, in response to a first user operation for any new first application card in the second interface, the electronic device may adjust a display order/display location of the new first application card. For example, the second interface is the user interface 540 shown in FIG. 6(A), and the electronic device may move, in response to a drag operation from bottom to top for the new first application card 542 in the user interface 540, a display location of the new first application card 542 to be above the new first application card 541. It may be understood that the electronic device adjusts a display order (a top-bottom display order) of application cards in the user interface 540: the new first application card 541, the new first application card 542, the new first application card 543, and the first application card 544 to a display order (a top-bottom display order) in the user interface 610: the new first application card 542, the new first application card 541, the new first application card 543, and the first application card 544.

S203: The electronic device displays a fourth interface (including application cards displayed in the adjusted display order).

In an implementation, the electronic device may display at least one new first application card and optionally at least one first application card in the fourth interface based on the adjusted display order, for example, display the user interface 610 shown in FIG. 6(B).

This is not limited to the foregoing implementation. In another implementation, in response to a user operation for any first application card in the second interface, the electronic device may adjust a display order/display location of the first application card. In another implementation, in response to a user operation for any second application card in the first application card, the electronic device may adjust a display order/display location of the second application card in the first application card.

In the method shown in FIG. 13, the user may set a display order of at least one new first application card based on a preference, so that the adjusted display order meets a user habit. This is convenient for the user to view information such as the measurement result of the physiological parameter, thereby further improving efficiency of obtaining information by the user. In addition, each first application card includes at least one second application card. In other words, the user can directly view a physiological category to which each second application card belongs, and can move a plurality of second application cards of a same physiological category at one time. Therefore, user operations are convenient.

FIG. 14 is a schematic flowchart of another display method according to an embodiment of this application. The method may be applied to the electronic device 100 shown in FIG. 1. The method may be applied to the electronic device 100 shown in FIG. 2. The method may be applied to the electronic device 200 shown in FIG. 3. The method may include but is not limited to the following steps.

S301: An electronic device receives a second user operation.

In an implementation, before S301 in FIG. 14, S101 to S104 in FIG. 12 are further included.

S302: The electronic device performs data fusion on measurement data corresponding to a plurality of new first application cards, to obtain fused result data.

In an implementation, the electronic device may perform, in response to the second user operation for the plurality of new first application cards, data fusion on the measurement data corresponding to the plurality of new first application cards, to obtain the fused result data. The fused result data may correspond to a new physiological parameter. In some examples, in response to a pinch operation for the new first application card 711 and the new first application card 712 in the user interface 710 shown in FIG. 7(A), the electronic device may perform data fusion on measurement data of the blood pressure corresponding to the new first application card 711 and measurement data of the sleep apnea corresponding to the new first application card 712, to obtain fused result data. This is not limited thereto. In some other examples, the electronic device may first respond to a touch operation for the new first application card 811 in the user interface 810 shown in FIG. 8A(A), then respond to a touch operation for the new first application card 812 in the user interface 820 shown in FIG. 8A(B), select the new first application card 811 and the new first application card 812, and perform data fusion on abnormal data in two types of data: the measurement data of the blood pressure corresponding to the new first application card 811 and the measurement data of the sleep apnea corresponding to the new first application card 812, to obtain the fused result data. For example, the electronic device detects that some data in the measurement data of the blood pressure is abnormal. It is assumed that measurement time corresponding to abnormal data 1 is 02:42 to 02:53. The electronic device may select, from the measurement data of the sleep apnea, data 2 corresponding to the measurement time 02:42 to 02:53, and perform data fusion on the data 1 and the data 2, to obtain fused data 3. The data 1 is, for example, data displayed in a gray area below "Blood pressure" in the user interface 830 shown in FIG. 8A(C). The data 2 is, for example, data displayed in a gray area below "Respiration" in the user interface 830. The fused data 3 is, for example, data displayed in a gray area below "Sympathetic nerve activity" in the user interface 830.

In an implementation, the electronic device may fuse the plurality of new first application cards, and display fused result data in a result application card. For example, the fused application card is the new first application card 721 in the user interface 720 shown in FIG. 7(C), and the fused result data is the result data of the OSA end-stage blood pressure.

S303: The electronic device displays a fifth interface (including the fused result data).

In an implementation, the electronic device may display a fifth interface. The fifth interface may include a fused application card, and the fused application card may include information of a new physiological parameter and fused result data. The fused result data may include but is not limited to information such as a measurement result of the new physiological parameter, generation time of the fused result data, and measurement time of the measurement data. In some examples, the fifth interface is the user interface 720 shown in FIG. 7(C), and the fused application card is the new first application card 721 in the user interface 720. The new first application card 721 may include information of the OSA end-stage blood pressure (namely, information indicated by a title "Blood pressure health study VS sleep apnea study" and the characters "OSA end-stage blood pressure" included in the text content 7211), a measurement result of the OSA end-stage blood pressure (namely, a result represented by the characters "220/110 mmHg" included in the text content 7212), and generation time of the result data of the OSA end-stage blood pressure (namely, the characters "8/14 02:24" included in the text content 7213).

This is not limited to the foregoing example. In some other examples, the fused application card may further include result data of original application cards (namely, result data corresponding to the plurality of new first application cards used for data fusion). For example, the fifth interface is the user interface 730 shown in FIG. 7(D), and the fused application card is the new first application card 731 in the user interface 730. The new first application card 731 is similar to the new first application card 721, and a difference lies in that the new first application card 731 further includes the result data of the blood pressure and the result data of the sleep apnea.

This is not limited to the foregoing implementation. In another implementation, a foldable electronic device may display the fused result data and the result data of the original application cards in a display area in the fifth interface. For example, the fifth interface is the user interface 830 shown in FIG. 8A(C). The display area 832 in the user interface 830 may include information of a new physiological parameter (namely, information indicated by the title "Blood pressure health study VS sleep apnea study" and the characters "Sympathetic nerve activity" included in the text content 8322), the fused result data (namely, the curve chart displayed below the text content 8322), the result data of the sleep apnea (namely, the curve chart displayed below the text content 8323), and the result data of the blood pressure (namely, the curve chart displayed below the text content 8324), and analysis data (namely, information indicated by characters "Result interpretation").

Optionally, after obtaining the fused result data, the electronic device may detect whether there is abnormal data in the fused result data. When detecting that there is abnormal data, the electronic device may highlight the abnormal data and related information (for example, a curve chart of the abnormal data, time corresponding to the abnormal data, and an analysis result of the abnormal data) in the fifth interface, to prompt the user.

In the method shown in FIG. 14, the electronic device may perform data fusion on measurement data of any plurality of types of physiological parameters, to generate fused result data. The fused result data is more comprehensive, a physical condition of the user can be further analyzed by using the fused result data, especially in a case in which the user may have an abnormal symptom, so that the user can better understand a health status of the user, thereby further improving user experience.

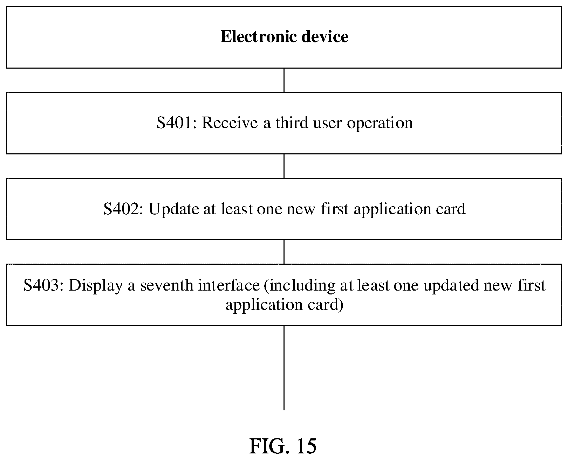

FIG. 15 is a schematic flowchart of another display method according to an embodiment of this application. The method may be applied to the electronic device 100 shown in FIG. 1. The method may be applied to the electronic device 100 shown in FIG. 2. The method may be applied to the electronic device 200 shown in FIG. 3. The method may include but is not limited to the following steps.

S401: An electronic device receives a third user operation.

In an implementation, before S401 in FIG. 15, S101 to S104 in FIG. 12 are further included.

S402: The electronic device updates at least one new first application card.

In an implementation, the electronic device may obtain, in response to the third user operation for a sixth interface, measurement data of a physiological parameter corresponding to at least one new first application card in the sixth interface, to update result data corresponding to the at least one new first application card. The sixth interface may be a user interface of a health application.

In an implementation, the electronic device may sequentially obtain, in a first preset order, measurement data of a plurality of types of physiological parameters corresponding to a plurality of new first application cards. In some examples, the first preset order may be a top-bottom display order of the plurality of new first application cards in the user interface of the application. It may also be understood that an order in which the electronic device obtains the measurement data of the plurality of types of physiological parameters corresponding to the plurality of new first application cards is consistent with the top-bottom display order of the plurality of new first application cards. In some examples, the sixth interface may be a home page of an application. For example, the sixth interface is the user interface 540 shown in FIG. 9A(A). In response to a sliding operation from top to bottom for the user interface 540, the electronic device may, sequentially obtain, in a first preset order (the top-bottom display order of the plurality of new first application cards in the user interface 540 is: the new first application card 541, the new first application card 542, and the new first application card 543), measurement data of the heart rate corresponding to the new first application card 541, measurement data of the lung function corresponding to the new first application card 542, and measurement data of the liver fat corresponding to the new first application card 543. In this case, the electronic device may display progress information, for example, display progress information represented by the control 912 and the text content 911 in the user interface 910 shown in FIG. 9A(B).

In some examples, a size of measurement data of a physiological parameter (for example, the measurement data of the sleep apnea and the measurement data of the respiration) usually ranges from tens of megabytes to hundreds of megabytes. When the electronic device updates the result data of the physiological parameter, because a transmission rate of Bluetooth is low, a rate at which the electronic device obtains data is also low, and the electronic device usually needs to wait for several minutes when the electronic device obtains the measurement data of the plurality of types of physiological parameters from a wearable device through Bluetooth. Therefore, the electronic device may sequentially obtain the plurality of types of physiological parameters in the first preset order, to improve orderliness of data transmission, thereby increasing a data transmission rate. Optionally, when obtaining the measurement data of the plurality of types of physiological parameters, the electronic device may preferentially obtain the data from the cloud or a related application. If the cloud does not store the data, the data may be first transmitted to the cloud by using the wearable device. After the cloud obtains the data, the electronic device obtains the data from the cloud, so that the data transmission rate is improved, and waiting time of the user is shortened.

This is not limited to the foregoing implementation. In another implementation, the sixth interface may be a detailed interface of any type of physiological parameter, and the electronic device may obtain, in response to a user operation for the detailed interface, measurement data of a physiological parameter corresponding to the detailed interface. For example, the sixth interface is the user interface 930 shown in FIG. 9B(A). The electronic device may obtain the measurement data of the liver fat in response to a sliding operation from top to bottom for the user interface 930. In this case, the electronic device may display progress information, for example, display the progress information represented by the text content 941 in the user interface 940 shown in FIG. 9B(B).

This is not limited to a case in which the electronic device obtains measurement data of a corresponding physiological parameter in a detailed interface of any type of physiological parameter. In some other examples, when the electronic device displays the detailed interface of any type of physiological parameter, and the electronic device is obtaining the measurement data of the physiological parameter, the electronic device may display, in the detailed interface in response to a user operation for the detailed interface, the progress information of obtaining the physiological parameter. For example, the sixth interface is the user interface 930 shown in FIG. 9B(A). In this case, the electronic device is obtaining the measurement data of the heart rate, and the electronic device may display progress information in response to a sliding operation from top to bottom for the user interface 930. The progress information includes, for example, the characters "Synchronizing data of heart health study (50%). Synchronize data of liver fat study later".

This is not limited to the foregoing implementation. In another implementation, when the health-type application is started, the electronic device may obtain measurement data of a physiological parameter corresponding to at least one new first application card, to update result data corresponding to the at least one new first application card. In some examples, in response to a touch operation for a control of the application, the electronic device may start the application, and sequentially obtain the measurement data of the heart rate, the measurement data of the lung function, and the measurement data of the liver fat in a first preset order (for example, the top-bottom display order of the plurality of new first application cards in the user interface 540 shown in FIG. 9A(A)). In this case, the electronic device may display progress information, for example, display the progress information represented by the control 1011 in the user interface 1010 shown in FIG. 10(A).

In an implementation, after obtaining the measurement data of the at least one type of physiological parameter corresponding to the at least one new first application card, the electronic device may separately analyze the at least one type of measurement data, to generate result data of the at least one type of physiological parameter, and display the at least one type of generated result data in the corresponding new first application card, to update the at least one new first application card.

S403: The electronic device displays a seventh interface (including at least one updated new first application card).

In an implementation, the electronic device may display the seventh interface. The seventh interface may include the at least one updated new first application card. In some examples, the user interface of the electronic device before the update is the user interface 540 shown in FIG. 9A(A). A user interface used when the electronic device obtains the measurement data of the physiological parameter is the user interface 910 shown in FIG. 9A(B). The seventh interface of the electronic device after the update is similar to the user interface 540, and a difference lies in that result data of three new first application cards in the seventh interface is different from result data of three new first application cards in the user interface 540.

This is not limited to the foregoing implementation. In another implementation, the electronic device may automatically update the result data of the physiological parameter, for example, update the result data of the physiological parameter at an interval of preset duration, and may display FIG. 10(A) or FIG. 10(B) during the update.

In the method shown in FIG. 15, the electronic device may automatically update/update, in response to a user operation, result data corresponding to at least one new first application card, and display progress information during the update to the user in a visualized manner, so that the user learns of an update progress status in time, thereby reducing anxiety of the user in a waiting process, and further improving user experience.

In the foregoing embodiment, an example in which the related information of the physiological parameter is displayed in a form of card is used for description. In some other embodiments, the related information of the physiological parameter may alternatively be displayed in a form of another control like a list box.

In the foregoing embodiment, an example in which any application card (the new first application card or the second application card) indicates one type of physiological parameter is used for description. In some other embodiments, the new first application card may further indicate at least one type of physiological parameter, and the second application card may indicate at least one type of physiological parameter.

All or some of the methods provided in embodiments of this application may be implemented by using software, hardware, firmware, or any combination thereof. When software is used to implement the methods, all or a part of embodiments may be implemented in a form of computer program product. The computer program product includes one or more computer instructions. When the computer program instructions are loaded and executed on the computer, the procedure or functions according to embodiments of this application are all or partially generated. The computer may be a general-purpose computer, a dedicated computer, a computer network, a network device, user equipment, or another programmable apparatus. The computer instructions may be stored in a computer-readable storage medium or may be transmitted from a computer-readable storage medium to another computer-readable storage medium. For example, the computer instructions may be transmitted from a website, computer, server, or data center to another website, computer, server, or data center in a wired (for example, a coaxial cable, an optical fiber, or a digital subscriber line (digital subscriber line, DSL)) or wireless (for example, infrared, radio, or microwave) manner. The computer-readable storage medium may be any usable medium accessible by the computer or a data storage device, for example, a server or a data center, integrating one or more usable media. The usable medium may be a magnetic medium (for example, a floppy disk, a hard disk drive, or a magnetic tape), an optical medium (for example, a digital video disc (digital video disc, DVD)), a semiconductor medium (for example, a solid state disk (solid state disk, SSD)), or the like. In conclusion, the foregoing embodiments are merely intended for describing the technical solutions in this application, but not for limiting this application. Although this application is described in detail with reference to the foregoing embodiments, persons of ordinary skill in the art should understand that they may still make modifications to the technical solutions recorded in the foregoing embodiments or make equivalent replacements to some technical features thereof. However, these modifications or replacements do not make the essence of the corresponding technical solutions depart from the scope of the technical solutions in embodiments of this application.

## Claims

1. A display method, applied to a first device, wherein a first application is installed on the first device, and the method comprises:
displaying a first interface of the first application, wherein the first interface comprises a first application card, a second application card is displayed in the first application card, and measurement information of a physiological parameter is not displayed in the second application card;
obtaining measurement information of at least one type of physiological parameter; and
displaying a second interface of the first application, wherein the second interface comprises a new first application card, the measurement information is displayed in the new first application card, the new first application card is displayed above the first application card, and the second application card is not displayed in the first application card.

2. The method according to claim 1, wherein the new first application card is displayed in the second interface in a first display order, and the first display order is related to at least one of the following: generation time of the new first application card, obtaining time of the measurement information of the physiological parameter indicated by the new first application card, and a risk level of the measurement information of the physiological parameter indicated by the new first application card.

3. The method according to claim 2, wherein the first display order is related to the risk level of the measurement information of the physiological parameter indicated by the new first application card, the new first application card comprises a third application card and a fourth application card, the third application card is displayed above the fourth application card, and a risk level of measurement information of a physiological parameter indicated by the third application card is higher than a risk level of measurement information of a physiological parameter indicated by the fourth application card.

4. The method according to any one of claims 1 to 3, wherein a plurality of application cards are displayed in the new first application card, and the method further comprises:
receiving a first operation for the plurality of application cards;
in response to the first operation, obtaining first information based on measurement information of a plurality of types of physiological parameters respectively indicated by the plurality of application cards; and
displaying a third interface of the first application, wherein the third interface comprises the first information.

5. The method according to claim 4, wherein the third interface comprises a fifth application card, the fifth application card indicates a first physiological parameter, the fifth application card comprises the first information, and the first physiological parameter comprises the plurality of types of physiological parameters respectively indicated by the plurality of application cards.

6. The method according to claim 5, wherein the fifth application card comprises the measurement information of the plurality of types of physiological parameters respectively indicated by the plurality of application cards, and the third interface does not comprise the plurality of application cards.

7. The method according to any one of claims 1 to 6, wherein the method further comprises:
receiving a second operation for the second interface;
obtaining second information in response to the second operation, wherein the second information comprises the measurement information that is of the physiological parameter indicated by the new first application card and that is obtained at first time, the first time is later than obtaining time of measurement information of a physiological parameter indicated by a sixth application card in the second interface, and the sixth application card is any application card in the new first application card;
displaying first progress information in response to the second operation, wherein the first progress information indicates progress of obtaining the second information; and
updating, based on the second information, the measurement information of the physiological parameter indicated by the new first application card in the second interface for display.

8. The method according to claim 7, wherein the obtaining second information comprises:
sequentially obtaining, in a first preset order, the measurement information of the physiological parameter indicated by the new first application card, wherein the first preset order is related to a display order of the new first application card in the second interface.

9. The method according to any one of claims 1 to 8, wherein the new first application card in the second interface indicates a second physiological parameter, and the method further comprises:
receiving a third operation for the new first application card;
displaying a fourth interface of the first application in response to the third operation, wherein the fourth interface comprises measurement information of the second physiological parameter;
receiving a fourth operation for the fourth interface;
in response to the fourth operation, if the first device is currently obtaining measurement information of a third physiological parameter, displaying progress of obtaining the measurement information of the third physiological parameter; and
in response to the fourth operation, if the first device currently does not obtain measurement information of a physiological parameter, obtaining third information, wherein the third information is the measurement information that is of the second physiological parameter and that is obtained at second time, and the second time is later than obtaining time of the measurement information of the second physiological parameter in the fourth interface; displaying second progress information, wherein the second progress information indicates progress of obtaining the third information; and updating, based on the third information, the measurement information of the second physiological parameter in the fourth interface for display.

10. The method according to any one of claims 1 to 9, wherein the method further comprises:
displaying a first card in a fifth interface, wherein the fifth interface is an interface of the first device other than an interface of the first application, and the first card is any application card in the new first application card.

11. The method according to claim 10, wherein the method further comprises:
receiving a fifth operation for the first card; and
switching the first card to a second card in response to the fifth operation, wherein the first card is different from the second card, and the new first application card comprises the second card.

12. The method according to any one of claims 1 to 11, wherein the new first application card in the second interface indicates the second physiological parameter, and the method further comprises:
receiving a sixth operation for the new first application card; and
displaying a fifth interface of the first application in response to the sixth operation, wherein the fifth interface does not comprise the new first application card, the fifth interface comprises the second application card, the second application card indicates the second physiological parameter, and the measurement information of the second physiological parameter is not displayed in the second application card.

13. The method according to any one of claims 1 to 12, wherein the obtaining measurement information of at least one type of physiological parameter comprises at least one of the following:
obtaining the measurement information of the at least one type of physiological parameter from a second application related to the at least one type of physiological parameter;
receiving the measurement information that is of the at least one type of physiological parameter and that is sent by a second device, wherein the measurement information of the at least one type of physiological parameter is obtained by the second device by measuring a user; and
obtaining the measurement information of the at least one type of physiological parameter from a cloud.

14. The method according to claim 13, wherein the second application card in the first interface indicates a fourth physiological parameter, and the obtaining measurement information of at least one type of physiological parameter comprises:
receiving a seventh operation for the second application card;
displaying a sixth interface of the first application in response to the seventh operation;
receiving an eighth operation for a first control in the sixth interface; and
obtaining measurement information of the fourth physiological parameter in response to the eighth operation.

15. An electronic device, comprising a transceiver, a processor, and a memory, wherein the memory is configured to store a computer program, and the processor invokes the computer program to perform the method according to any one of claims 1 to 14.

16. A computer storage medium, wherein the computer storage medium stores a computer program, and when the computer program is executed by a processor, the method according to any one of claims 1 to 14 is implemented.
